# EUROPEAN PATENT APPLICATION

(11) **EP 4 537 857 A1**
(43) Date of publication of application: **16.04.2025**
(21) Application number: 22945917.7
(22) Date of filing: 04.10.2022
(51) Int. Cl.: A61L 9/12, G01N 1/00

(54) **CARTRIDGE FOR ODOR GENERATION DEVICE, ODOR GENERATION DEVICE, AND OLFACTORY TEST DEVICE**

(30) Priority: 08.06.2022 JP 2022093121
(71) Applicant: Sony Group Corporation, Tokyo 108-0075 (JP)
(72) Inventor: INOUE, Yukito, Tokyo 108-0075 (JP); FUJITA, Shuji, Tokyo 108-0075 (JP); TAKAKI, Kazutaka, Tokyo 108-0075 (JP); KIHARA, Hirotaka, Tokyo 108-0075 (JP); TSUJI, Kazuhiko, Tokyo 108-0075 (JP)
(74) Representative: D Young & Co LLP
(86) International application number: PCT/JP2022/037191
(87) International publication number: WO 2023/238421

(57) **Abstract**

To provide a technology capable of preventing odor leakage.

There is provided a cartridge for an odor generation device, the cartridge including: an odor retaining portion configured to retain an odor component; a ventilation portion having a ventilation opening that is openable and closable; and a connecting portion including at least two connection openings connecting the ventilation portion and the odor retaining portion, and a partition disposed upstream of one connection opening among the at least two connection openings. Furthermore, there are also provided an odor generation device and an olfactory examination device including the cartridge and a cartridge holding unit.

## Description

### TECHNICAL FIELD

The present technology relates to a cartridge for an odor generation device, an odor generation device, and an olfactory examination device.

### BACKGROUND ART

In the related art, a technique has been proposed in which air is supplied to a storage device in which an odor component holding member that holds an odor component is stored, and a vaporized odor component is released by a flow of air.

For example, Patent Document 1 proposes a scent dispensing device including: a cylindrical shielding tube whose internal cavity serves as a fragrance transport airway and that has an opening on a part of a side surface; a fragrance container that stores a fragrance inside, is provided on an outer surface of the shielding tube, and has an opening on a side surface on the shielding tube side; and a blowing means capable of blowing air to one end of the fragrance transport airway, in which the interior of the fragrance container and the fragrance transport airway can be brought into a penetrating state by overlapping the opening of the shielding tube with the opening of the fragrance container through a relative rotational movement while the shielding tube and the fragrance container are in close contact with each other, and the interior of the fragrance container and the fragrance transport airway can be brought into a non-penetrating state by overlapping one opening of the shielding tube and the fragrance container with a portion other than the one opening through a relative rotational movement while the shielding tube and the fragrance container are in close contact with each other.

According to the scent dispensing device described in Patent Document 1, unintended release of the fragrance can be suppressed, and scent can be dispensed at an appropriate timing.

### CITATION LIST

### PATENT DOCUMENT

Patent Document 1: Japanese Patent Application Laid-Open No. 2013-094436

### SUMMARY OF THE INVENTION

### PROBLEMS TO BE SOLVED BY THE INVENTION

However, in the conventional scent dispensing device, it is conceivable that the fragrance leaks from a fitting portion between the container holding the fragrance and a case, or an opening such as a scent entrance connected to the outside. As a result, problems may arise such as deterioration (that is, weakening due to volatilization, alteration due to oxidation, and the like) of the fragrance, mixing of the fragrance, and dissolution of a label attached to the case or the like.

Therefore, a main object of the present technology is to provide a technology capable of preventing odor leakage.

### SOLUTIONS TO PROBLEMS

That is, first, the present technology provides a cartridge for an odor generation device, the cartridge including: an odor retaining portion configured to retain an odor component; a ventilation portion having a ventilation opening that is openable and closable; and a connecting portion including at least two connection openings connecting the ventilation portion and the odor retaining portion, and a partition disposed upstream of one connection opening among the at least two connection openings.

Furthermore, the present technology also provides an odor generation device including: a cartridge for an odor generation device, the cartridge including an odor retaining portion configured to retain an odor component, a ventilation portion having a ventilation opening that is openable and closable and a connecting portion including at least two connection openings connecting the ventilation portion and the odor retaining portion, and a partition disposed upstream of one connection opening among the at least two connection openings; and a cartridge holding unit configured to hold the cartridge for the odor generation device.

Moreover, the present technology also provides an olfactory examination device including: a cartridge for an odor generation device, the cartridge including an odor retaining portion configured to retain an odor component, a ventilation portion having a ventilation opening that is openable and closable and a connecting portion including at least two connection openings connecting the ventilation portion and the odor retaining portion, and a partition disposed upstream of one connection opening among the at least two connection openings; and a cartridge holding unit configured to hold the cartridge for the odor generation device.

### BRIEF DESCRIPTION OF DRAWINGS

Figs. 1A to 1F are six views illustrating an embodiment of a cartridge 10 for an odor generation device according to a first embodiment.
Fig. 2 is a perspective view illustrating an embodiment of the cartridge 10 for an odor generation device according to the first embodiment.
Fig. 3 is a cross-sectional view taken along line P-P in the embodiment illustrated in Fig. 1.
Figs. 4A and 4B are views illustrating an embodiment of an odor retaining portion 101.
Fig. 5 is a cross-sectional view taken along line P-P of an embodiment different from the embodiment illustrated in Fig. 1.
Fig. 6 is a view illustrating an embodiment of a fixing portion 23.
Fig. 7 is a cross-sectional view taken along line P-P in the embodiment illustrated in Fig. 1.
Fig. 8 is a perspective view illustrating an embodiment different from the embodiment illustrated in Figs. 1 and 5.
Fig. 9 is a cross-sectional view taken along line P-P in the embodiment illustrated in Fig. 1.
Fig. 10 is a cross-sectional view illustrating an embodiment of a locking portion 51.
Fig. 11 is a conceptual cross-sectional view illustrating a state in which an odor is released from the cartridge 10 for an odor generation device according to the first embodiment.
Fig. 12 is a perspective conceptual view illustrating a state in which the cartridge 10 for an odor generation device according to the first embodiment is manufactured.
Fig. 13 is a perspective conceptual view illustrating a state in which the cartridge 10 for an odor generation device according to the present technology is manufactured using a dedicated jig Y.
Fig. 14 is a perspective conceptual view illustrating a state in which the cartridge 10 for an odor generation device according to the present technology is manufactured using the dedicated jig Y.
Fig. 15 is a perspective conceptual view illustrating a state in which the cartridge 10 for an odor generation device according to the present technology is manufactured using the dedicated jig Y.
Fig. 16 is an exploded perspective view illustrating an embodiment of an odor generation device 1 according to a fourth embodiment.
Fig. 17 is an exploded perspective view illustrating an embodiment of a cartridge holding unit 11.
Fig. 18 is a perspective view illustrating an embodiment of an indwelling portion 111.
Fig. 19 is a flowchart for explaining an operation example of an olfactory examination or olfactory training.
Fig. 20 is six views illustrating an embodiment of a cartridge 10 for an odor generation device according to a second embodiment.
Fig. 21 is a perspective view illustrating an embodiment of the cartridge 10 for an odor generation device according to the second embodiment.
Fig. 22 is a cross-sectional view taken along line P-P in the embodiment illustrated in Fig. 20.
Fig. 23 is a cross-sectional view taken along line P-P in the embodiment illustrated in Fig. 20.
Fig. 24 is a conceptual cross-sectional view illustrating a state in which an odor is released from the cartridge 10 for an odor generation device according to the second embodiment.
Fig. 25 is a view illustrating a state in which an outer layer portion 212 is removed and an odor retaining portion 101 is viewed from directly above.
Fig. 26 is a view illustrating a state in which a lid 22 of the odor retaining portion 101 is removed.
Fig. 27 is an enlarged view of a main part of a nozzle-shaped portion 1101 in an odor generation device 1 according to a fifth embodiment.
Fig. 28 is an exploded perspective view illustrating an embodiment of the odor generation device 1 according to the fifth embodiment.
Fig. 29 is a cross-sectional view illustrating an embodiment of a cartridge 10 for an odor generation device according to a third embodiment.
Fig. 30 is a conceptual cross-sectional view illustrating a state in which an odor is released from the cartridge 10 for an odor generation device according to the third embodiment.
Fig. 31 is a conceptual cross-sectional view illustrating a state in which an odor is released from the cartridge 10 for an odor generation device according to the third embodiment.

### MODE FOR CARRYING OUT THE INVENTION

Hereinafter, preferred modes for carrying out the present technology will be described with reference to the drawings.

Embodiments to be described hereinafter illustrate examples of representative embodiments of the present technology, and any embodiments can be combined. Furthermore, the scope of the present technology is not narrowly construed based on these. Note that the description is given in the following order.
1. First Embodiment (Cartridge 10 for Odor Generation Device)
   (1) Overall configuration
   (2) Odor retaining portion 101
   (3) Ventilation portion 102
   (4) Connecting portion 103
   (5) Releasing portion 104
   (6) Operation example of cartridge 10 of first embodiment
   (7) Manufacturing example of cartridge 10 of first embodiment
2. Second Embodiment (Cartridge 10 for Odor Generation Device)
   (1) Overall configuration
   (2) Odor retaining portion 101
   (3) Ventilation portion 102
   (4) Connecting portion 103
   (5) Operation example of cartridge 10 of second embodiment
   (6) Manufacturing example of cartridge 10 of second embodiment
3. Third Embodiment (Cartridge 10 for Odor Generation Device)
   (1) Overall configuration
   (2) Odor retaining portion 101
   (3) Ventilation portion 102
   (4) Connecting portion 103
   (5) Operation example of cartridge 10 of second embodiment
   (6) Manufacturing example of cartridge 10 of second embodiment
4. Fourth Embodiment (Odor Generation Device 1)
   (1) Overall configuration
   (2) Cartridge holding unit 11
   (3) Front storage portion 12
   (4) Back storage portion 13
   (5) Operation example of odor generation device 1 of fourth embodiment
   (6) Usage example of odor generation device 1 of fourth embodiment
5. Fifth Embodiment (Odor Generation Device 1)
   (1) Overall configuration
   (2) Cartridge holding unit 11
   (3) Front storage portion 12
   (4) Back storage portion 13
   (5) Operation example of odor generation device 1 of fifth embodiment
   (6) Usage example of odor generation device 1 of fifth embodiment

### 1. First Embodiment (Cartridge 10 for Odor Generation Device)

### (1) Overall configuration

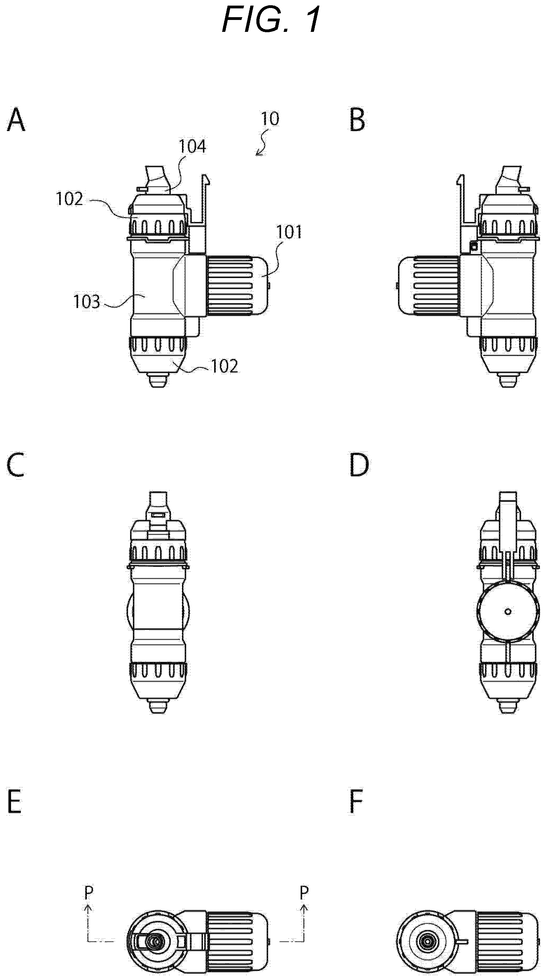

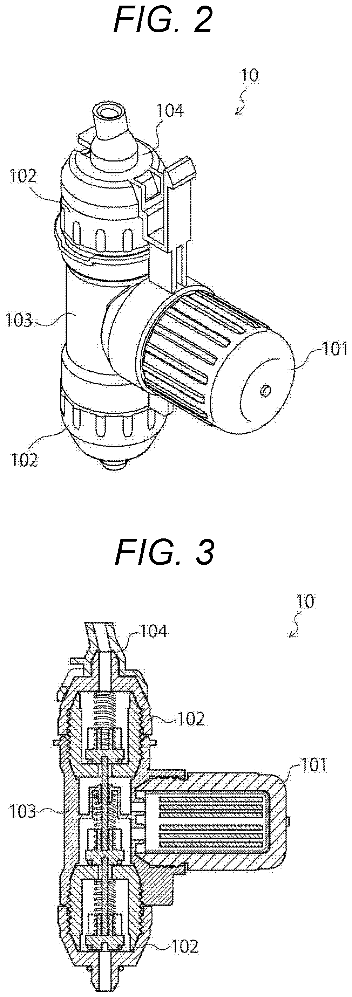

Fig. 1 is six views illustrating an embodiment of a cartridge 10 for an odor generation device (hereinafter, also referred to as a "cartridge 10") according to a first embodiment, in which A is a front view, B is a rear view, C is a left side view, D is a right side view, E is a plan view, and F is a bottom view. Furthermore, Fig. 2 is a perspective view illustrating an embodiment of the cartridge 10 for an odor generation device according to the first embodiment, and Figs. 3, 7, and 9 are cross-sectional views taken along line P-P in the embodiment illustrated in Fig. 1.

The cartridge 10 includes at least an odor retaining portion 101, a ventilation portion 102, and a connecting portion 103. Furthermore, a releasing portion 104 and the like may also be provided as necessary.

Hereinafter, details of each portion of the cartridge 10 of the first embodiment will be described.

### (2) Odor retaining portion 101

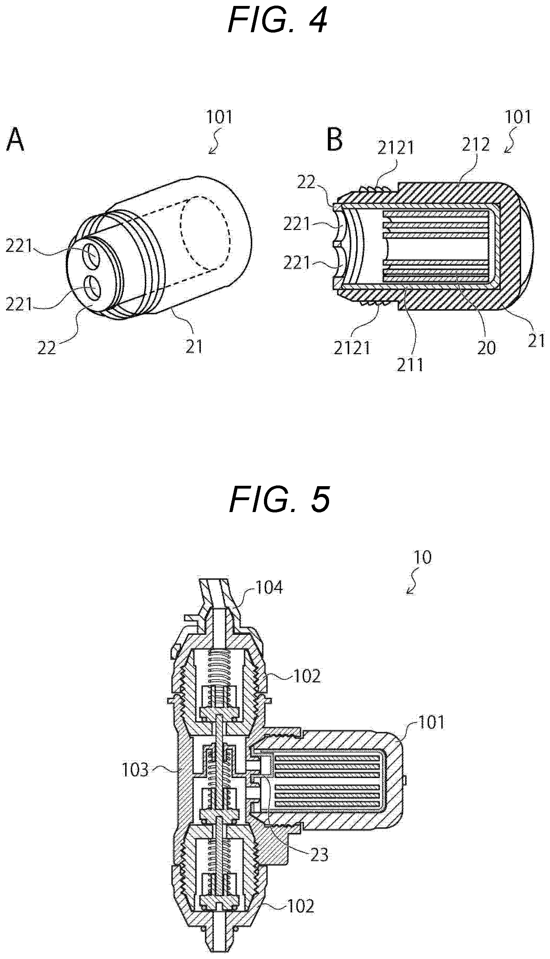

Fig. 4 is a view illustrating an embodiment of the odor retaining portion 101, in which A is a perspective view and B is a schematic cross-sectional view. The cartridge 10 includes the odor retaining portion 101 configured to retain an odor and the odor retaining portion 101 includes at least an impregnant 20, a container portion 21, and a lid 22 in the present embodiment.

Note that, in the present specification, the "odor component" can include any component that stimulates a part or all of the receptors present in the nasal cavity, such as odor molecules. In the nasal cavity, besides olfactory receptors, receptors of the trigeminal nerve that control stimulation such as cold, hot, and pain exist, and the odor component in the present specification is a broad concept including all components that stimulate some or all of these receptors. Specifically, for example, in a case where menthol is used as an odor component, menthol can serve as a stimulus through an olfactory receptor as well as a cold stimulus through a receptor of the trigeminal nerve (TRPA1 channel).

A material forming the impregnant 20 is not particularly limited as long as an odor component can be retained. Specifically, the impregnant 20 contains, for example, an organic polymer material so that the odor component easily infiltrates. As the organic polymer material, for example, it is possible to use polyvinyl chloride, polyethylene, a phenol resin, an olefin resin, nylon, polyester, a synthetic rubber, a silicone resin, a natural rubber, a protein, a nucleic acid, a lipid, polysaccharide, or the like, or a material obtained by combining two or more of them. Furthermore, in addition to this, for example, it is also possible to use a polymer resin such as an acrylic resin, a urethane resin, an ABS resin, a polyether ether ketone (PEEK) resin, a polyacetal (POM) resin, a fluororesin, a cycloolefin polymer resin, or a polyimide resin, a metal such as stainless steel or aluminum, an inorganic crystal such as quartz, glass, filter paper, or the like, or a material obtained by combining two or more of them.

Furthermore, the impregnant 20 may be formed porous, and for example, a mesh structure, cork, mesoporous silica, calcium carbonate, or the like can be used. Note that the impregnant 20 may have a fiber structure or a layered structure (for example, clay minerals and the like) other than the porous structure. Note that, in the present embodiment, the impregnant 20 preferably has a structure having sufficient voids. As a result, an airflow can pass through a gap in the impregnant 20, and odor component-containing air can be efficiently generated. Furthermore, the impregnant 20 may be formed using ceramic or the like.

Examples of a form of the impregnant 20 include, but not particularly limited to, a sheet shape, a mesh shape, a strip shape (also including a dense body thereof), a particle state (also including a dense body thereof), a gel state, a liquid state (also including liquid maintained at surface tension, such as carriers), a foam state, a three-dimensional structure (for example, a pinholder shape, a spiral shape, a spring shape, or the like), a string state (also including a dense body thereof), and the like, and a sheet shape is preferable. In a case where the impregnant 20 is formed in a sheet shape, for example, the impregnant 20 can be rolled up in a cylindrical shape or in multiple layers to be stored in the container portion 21, but the present technology is not limited thereto as long as the impregnant 20 can be stored in the container portion 21. In a case where the impregnant 20 has a sheet shape, specific dimensions can be designed to be, for example, 10 to 80 mm in length, 5 to 20 mm in width, and the like.

It is preferable that the impregnant 20 is not packed near an inner wall (specifically, a side wall, a bottom surface, and the like) of the container portion 21. By leaving a gap, an airflow having flowed into the container portion 21 flows around the impregnant 20 as much as possible before flowing out, and odor component-containing air can be efficiently generated.

The odor component retained in the impregnant 20 is not particularly limited. As long as the component generates an odor, for example, such as a liquid fragrance, a powder fragrance, or the like as it is, or dissolved or dispersed in an appropriate solvent, an essential oil, an essential oil diluted with an appropriate solvent, fruit juice, a beverage, a food, a food dissolved or dispersed in an appropriate solvent, or the like, one or two or more of them can be freely combined and used.

The container portion 21 preferably has a two-layer structure, and includes an inner layer portion 211 forming an inner side for holding the impregnant 20 and an outer layer portion 212 forming an outer side of the container portion 21, in the present embodiment.

A material for forming the inner layer portion 211 is not particularly limited as long as the impregnant 20 can be held, but a part or the whole of the inner layer portion 211 is preferably formed containing glass or metal, in the present embodiment Examples of the metal include stainless steel (steel use stainless (SUS)), aluminum, and the like. Furthermore, in the present embodiment, an inner wall of the inner layer portion 211 on the impregnant 20 side may be formed using a metal foil, or the inner wall may be metal-plated.

A form of the inner layer portion 211 is not particularly limited as long as an odor component can be retained, and can be freely designed. Examples of the form include a cylindrical body, a polygonal tubular body having a polygonal (triangular, square and so on) cross-shape, a conical body, a polygonal cone body having a polygonal (triangular, square and so on) cross-shape, a shape obtained by freely combining one or two or more of them, or the like. Examples of specific dimensions to be designed in a case where the inner layer portion 211 is formed in a cylindrical body include, for example, a length of 5 to 50 mm, an inner diameter of 4 to 48 mm, an outer diameter of 5 to 50 mm, and the like.

A material for forming the outer layer portion 212 is not particularly limited, and it is possible to use, for example, a resin such as polyvinyl chloride, polyethylene, a phenol resin, an olefin resin, an acrylic resin, a urethane resin, an ABS resin, a polyether ether ketone (PEEK) resin, a polyacetal (POM) resin, a fluororesin, a cycloolefin polymer resin, or a polyimide resin, or a material obtained by combining two or more of them.

Similarly to the inner layer portion 211, a form of the outer layer portion 212 is not particularly limited as long as an odor component can be retained, and can be freely designed. Examples of the form include a cylindrical body, a polygonal tubular body having a polygonal (triangular, square and so on) cross-shape, a conical body, a polygonal cone body having a polygonal (triangular, square and so on) cross-shape, a shape obtained by freely combining one or two or more of them, or the like. Note that, in the present embodiment, the inner layer portion 211 and the outer layer portion 212 preferably have the same form. Examples of specific dimensions to be designed in a case where the outer layer portion 212 is formed in a cylindrical body include, for example, a length of 5 to 50 mm, an inner diameter of 4 to 48 mm, an outer diameter of 5 to 50 mm, and the like.

The outer layer portion 212 preferably includes a first fitting portion 2121 to be fitted to the connecting portion 103 described later. As a result, a sealing property can be improved.

A form of the first fitting portion 2121 is not particularly limited as long as the first fitting portion 2121 can be fitted to the connecting portion 103, but for example, as illustrated in B of Fig. 4, a protruding screw formed in a fitting region can be used. Note that, in addition to the above, a form may be employed in which a conventionally known fitting mechanism using a claw, a pin, a hook, a clamp, slide engagement, or the like is formed.

The lid 22 preferably has an opening 221 at a position corresponding to a connection opening 40 described later. As a result, it is possible to efficiently perform inflow of air into the container portion 21 and release of odor component-containing air to the outside of the container portion 21. Note that, in the present embodiment, a plurality of openings 221 is more preferably formed so as to correspond to the number of connection openings 40 to be described later.

A material for forming the lid 22 is not particularly limited, and it is possible to use, for example, a resin such as polyvinyl chloride, polyethylene, a phenol resin, an olefin resin, an acrylic resin, a urethane resin, an ABS resin, a polyether ether ketone (PEEK) resin, a polyacetal (POM) resin, a fluororesin, a cycloolefin polymer resin, or a polyimide resin, glass, metal such as stainless steel (steel use stainless (SUS)) or aluminum, or the like or a material obtained by combining two or more of them.

A form of the lid 22 can be freely designed according to the form of the inner layer portion 211 and the outer layer portion 212 described above. Examples of a specific dimension of the lid 22 to be designed in a case where the inner layer portion 211 and the outer layer portion 212 are formed in a cylindrical body include, for example, a diameter of 5 to 30 mm or the like.

Fig. 5 is a cross-sectional view taken along line P-P of an embodiment different from the embodiment illustrated in Fig. 1, of the cartridge 10 for an odor generation device according to the present technology. In the embodiment illustrated in Fig. 5, the odor retaining portion 101 further includes a fixing portion 23 that fixes movement of the inner layer portion 211. By including the fixing portion 23, it is possible to prevent the inner layer portion 211 from moving up and down or the like inside the outer layer portion 212 due to vibration, impact, or the like.

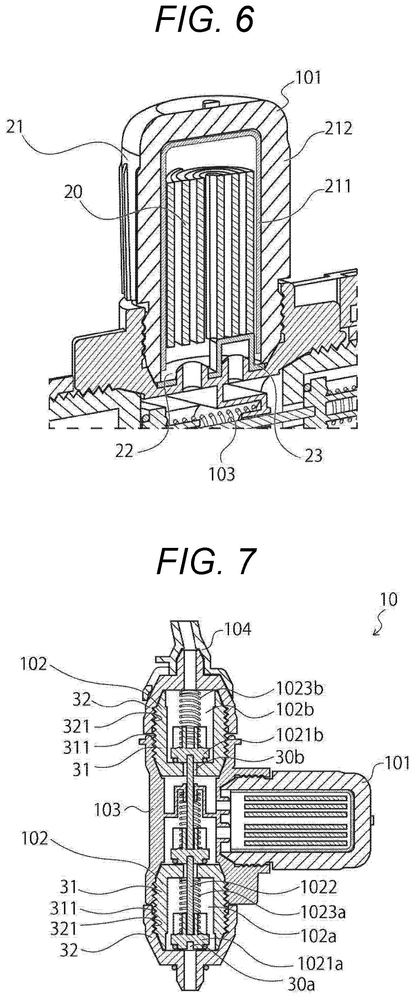

A form of the fixing portion 23 can be freely designed as long as movement of the inner layer portion 211 can be fixed, in accordance with the form of the inner layer portion 211 and the outer layer portion 212 described above. Specifically, for example, as illustrated in Fig. 6, a substantially U-shaped form formed in a part of the lid 22 can be adopted. In this case, the fixing portion 23 may be formed so as to press an upper end of the impregnant 20 in a state in which a free portion is directed to the connecting portion 103 side. Depending on a position and a storage state of the cartridge 10, it is conceivable that the top and bottom of the odor retaining portion 101 are reversed, the impregnant 20 itself moves, or dripping or the like occurs from the impregnant 20. However, by forming the fixing portion 23 in the form illustrated in Fig. 6, it is possible to prevent movement of the impregnant 20, dripping from the impregnant 20, and the like.

A material for forming the fixing portion 23 is not particularly limited, and for example, stainless steel (steel use stainless (SUS)), metal such as aluminum, or the like, or a combination of two or more of them can be used.

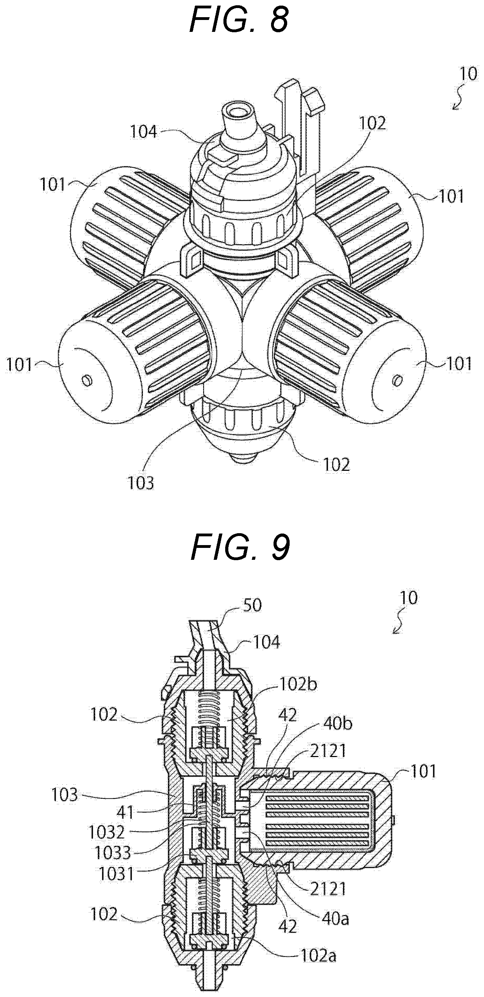

Fig. 8 is a perspective view illustrating an embodiment different from the embodiment illustrated in Figs. 1 and 5 of the cartridge 10 for an odor generation device according to the present technology. In the embodiment illustrated in Figs. 1 and 5, the cartridge 10 includes one odor retaining portion 101, but the present technology is not limited thereto. That is, as illustrated in Fig. 8, the cartridge 10 may include two or more odor retaining portions 101. In the embodiment illustrated in Fig. 8, by including two or more odor retaining portions 101, it is possible to increase a capacity of an odor component or store two or more types of odor components.

### (3) Ventilation portion 102

The ventilation portion 102 includes at least an openable and closable ventilation opening 30. In the present embodiment, as illustrated in Fig. 7, the ventilation portion 102 has a configuration divided into two sections (a first ventilation portion 102a and a second ventilation portion 102b) by the two ventilation openings 30 (30a, 30b), but the present technology is not limited thereto. That is, the ventilation portion 102 may be configured as a single section or may be configured to be divided into three or more sections.

In the embodiment illustrated in Fig. 7, the ventilation portion 102 has a symmetrical structure divided into two sections (the first ventilation portion 102a and the second ventilation portion 102b). With such a structure, components forming both sections can be shared, and cost reduction can be achieved.

In the present embodiment, the ventilation opening 30 includes an inflow ventilation opening 30a for allowing air to flow into the cartridge 10 and a release ventilation opening 30b for releasing odor component-containing air.

Furthermore, an opening and closing mechanism can be connected to these ventilation openings 30 (30a, 30b). A specific structure of the opening and closing mechanism is not particularly limited as long as the ventilation opening 30 (30a, 30b) can be opened and closed, and can be freely designed. Specifically, for example, an opening and closing mechanism including a sealing lid 1021 (1021a, 1021b), a shaft 1022, and a spring 1023 (1023a, 1023b) may be provided.

In the present embodiment, the ventilation portion 102 preferably has a two-layer structure, and includes an inner layer member 31 that stores an opening and closing mechanism, and an outer layer member 32 forming an outside of the ventilation portion 102, in the present embodiment.

In the present embodiment, as illustrated in Fig. 7, the inner layer member 31 includes a second fitting portion 311 in a fitting region between the outer layer member 32 and the connecting portion 103 to be described later. As a result, a sealing property can be improved. Furthermore, in this case, the outer layer member 32 preferably includes a second fitted portion 321 to be fitted to the second fitting portion 311 in a fitting region between with the inner layer member 31.

A form of the second fitting portion 311 is not particularly limited as long as the second fitting portion 311 can be fitted to the second fitted portion 321, but for example, as illustrated in Fig. 7, a protruding screw formed in the fitting region can be used. In this case, the second fitted portion 321 is preferably a recessed screw. Note that, in addition to the above, a form may be employed in which a conventionally known fitting mechanism using a claw, a pin, a hook, a clamp, slide engagement, or the like is formed.

### (4) Connecting portion 103

The connecting portion 103 includes: at least two connection openings 40 (40a, 40b) connecting the ventilation portion 102 and the odor retaining portion 101; and a partition 41 disposed upstream of one connection opening among the at least two connection openings 40 (40a, 40b). In the present embodiment, as illustrated in Fig. 9, the connection opening 40 includes the two connection openings 40 (40a, 40b), but the present technology is not limited thereto. That is, as illustrated in Fig. 8, in a case where the cartridge 10 includes a plurality of odor retaining portions 101, three or more connection openings 40 may be provided. Furthermore, as illustrated in Fig. 9, the connecting portion 103 may be configured as a single section or may be configured to be divided into two or more sections.

In the present embodiment, the two connection openings 40 (40a, 40b) preferably include: a first connection opening 40a configured to release air from the connecting portion 103 to the odor retaining portion 101; and a second connection opening 40b configured to release odor component-containing air from the odor retaining portion 101 to the connecting portion 103. In this case, the air having flowed into the connecting portion 103 via the first ventilation portion 102a is released to the odor retaining portion 101 via the first connection opening 40a and is mixed with the odor component, and thereafter, the odor component-containing air flows into the connecting portion 103 via the second connection opening 40b and is released to the outside via the second ventilation portion 102b.

Furthermore, in this case, a diameter of the first connection opening 40a is preferably smaller than a diameter of the second connection opening 40b. As a result, air can be strongly applied to the impregnant 20, and the odor component can be efficiently released.

In the present embodiment, the partition 41 is disposed upstream of the second connection opening 40b among the two connection openings 40 (40a, 40b). By including the partition 41, air having flowed into the connecting portion 103 forcibly becomes odor component-containing air via the odor retaining portion 101, and flows into the connecting portion 103 again. As a result, odor component-containing air can be efficiently generated.

Furthermore, in the present embodiment, the connecting portion 103 may include an opening and closing mechanism in a connection region between with the ventilation portion 102. Specifically, an opening and closing mechanism similar to that of the ventilation portion 102 can be used, and for example, an opening and closing mechanism including a sealing lid 1031, a shaft 1032, and a spring 1033 may be provided. In this case, while the shaft 1032 performs an operation of pushing from the upstream side of the cartridge 10, the partition 41 is preferably configured not to hinder the operation of the shaft 1032 as illustrated in Fig. 9. Note that, since the operation of the shaft 1032 itself has a high pressure loss, it is considered that an airflow does not pass even if a gap corresponding to the shaft 1032 is formed in a part of the partition 41 as illustrated in Fig. 9.

As illustrated in Fig. 9, the connecting portion 103 preferably includes a first fitted portion 42 to be fitted to the above-described first fitting portion 2121 in a fitting region between the odor retaining portion 101 and the ventilation portion 102. As a result, a sealing property can be improved. Note that, in a case where the form of the first fitting portion 2121 is a protruding screw formed in the fitting region, the first fitted portion 42 is preferably a recessed screw.

### (5) Releasing portion 104

As illustrated in Fig. 9, the releasing portion 104 has a nozzle structure 50 that can release odor component-containing air to the outside and change a direction of the odor component-containing air. The releasing portion 104 is not an essential configuration in the present technology, but including the releasing portion 104 makes it possible to uniformly discharge the odor component-containing air to a nose tip of a user by adjusting a releasing angle of a tip of the cartridge 10.

A form of the releasing portion 104 is not particularly limited, but can be a cap that covers the outer layer member 32, for example. As a result, a position of the nozzle structure 50 is not affected by a rotation tightening degree of the outer layer member 32 or the like. In a case where the releasing portion 104 is made as a cap, by adopting thread cutting, separation, and a chucking method necessary for sealing, it is not necessary to form a complicated mounting shape on a main body side of the cartridge 10, and the cap itself can have a mounting function. Note that the present technology is not limited thereto, and the outer layer member 32 and the releasing portion 104 may be integrally formed.

A material for forming the releasing portion 104 is not particularly limited, and it is possible to use, for example, a resin such as polyvinyl chloride, polyethylene, a phenol resin, an olefin resin, an acrylic resin, a urethane resin, an ABS resin, a polyether ether ketone (PEEK) resin, a polyacetal (POM) resin, a fluororesin, a cycloolefin polymer resin, or a polyimide resin, or a material obtained by combining two or more of them.

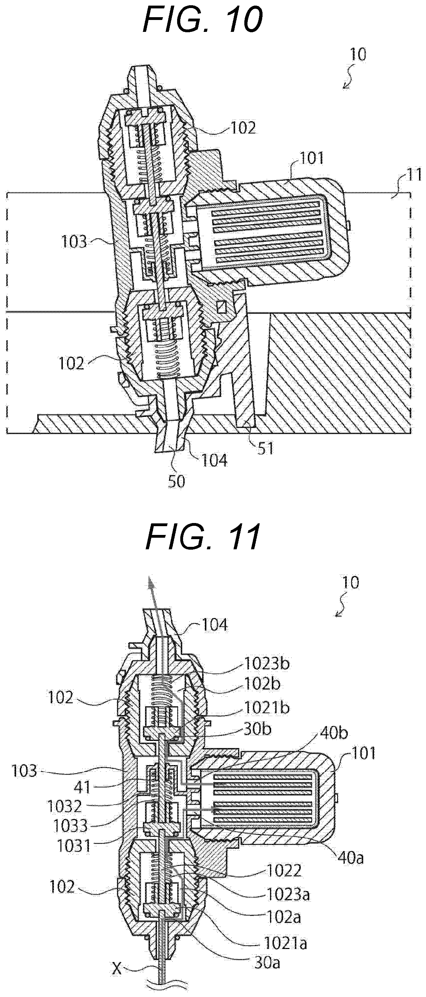

Furthermore, in the present embodiment, as illustrated in Fig. 10, the releasing portion 104 may include a locking portion 51 to be engaged with a part of a cartridge holding unit 11 to be described later. As a result, positioning, insertion, and the like of the cartridge 10 to the cartridge holding unit 11 can be easily performed. A form of the locking portion 51 is not particularly limited, and can be a claw shape, for example, as illustrated in Fig. 10.

### (6) Operation example of cartridge 10 of first embodiment

Fig. 11 is a conceptual cross-sectional view illustrating a state in which an odor is released from the cartridge 10 of the first embodiment. Hereinafter, with reference to Fig. 11, an operation example of releasing odor component-containing air in the cartridge 10 will be described.

In the embodiment illustrated in Fig. 11, when the ventilation portion 102 is opened in a state where an odor component is retained in the odor retaining portion 101, air flows in from the outside. The air having flowed in flows into the connecting portion 103, is mixed with the odor component through the one connection opening 40a, and is released as odor component-containing air. In this state, the released odor component-containing air flows into the connecting portion 103 via the another connection opening 40b, and is thereby released to the outside from the releasing portion 104. As a result, in the present embodiment, it is possible to efficiently generate odor component-containing air while preventing odor leakage due to unintended inflow of air or the like.

Specifically, in the present embodiment, the ventilation opening 30 (30a, 30b) is provided with the opening and closing mechanism including the sealing lid 1021 (1021a, 1021b), the shaft 1022, and the spring 1023 (1023a, 1023b), and the connecting portion 103 is also provided with the opening and closing mechanism including the sealing lid 1031, the shaft 1032, and the spring 1033. These opening and closing mechanisms can be controlled, for example, by inserting a pusher X from the odor generation device side or the like, from a lower portion of the cartridge 10. Note that, in Fig. 11, the sealing lid and the spring are provided for each of the three sections (the first ventilation portion 102a, the connecting portion 103, and the second ventilation portion 102b), but the present technology is not limited thereto, and the sealing lid and the spring may be provided only for some sections.

When the pusher X is pressed, the pusher X pushes a first sealing lid 1021a in an inward direction of the first ventilation portion 102a, the inflow ventilation opening 30a is opened, and air flows into the first ventilation portion 102a. At this time, a first spring 1023a is contracted by the first sealing lid 1021a.

When the first sealing lid 1021a is pushed in the inward direction of the first ventilation portion 102a, the shaft 1022 mounted to the first sealing lid 1021a moves in a direction of the connecting portion 103. The shaft 1022 pushes the sealing lid 1031 in an inward direction of the connecting portion 103, and air in the first ventilation portion 102a flows into the connecting portion 103. At this time, the spring 1033 is contracted by the sealing lid 1031.

Since there is the partition 41, the air having flowed into the connecting portion 103 first flows into the odor retaining portion 101 through the first connection opening 40a and is mixed with the odor component retained in the odor retaining portion 101, and odor component-containing air is generated. The odor component-containing air flows into the connecting portion 103 again through the second connection opening 40b.

When the sealing lid 1031 is pushed in the inward direction of the connecting portion 103, the shaft 1032 mounted to the sealing lid 1031 moves in a direction of the second ventilation portion 102b. The shaft 1032 pushes a second sealing lid 1021b in an inward direction of the second ventilation portion 102b, and the odor component-containing air in the connecting portion 103 flows into the second ventilation portion 102b. At this time, a second spring 1023b is contracted by the second sealing lid 1021b.

In the present embodiment, since the second ventilation portion 102b is connected to the release ventilation opening 30b, the odor component-containing air having flowed into the second ventilation portion 102b flows into the releasing portion 104 through the release ventilation opening 30b and is released to the outside.

When the pressing to the pusher X is released after the odor component-containing air is released to the outside, the first sealing lid 1021a is returned to an original position by a restoring force of the contracted first spring 1023a. Furthermore, the sealing lid 1031 is returned to an original position by a restoring force of the spring 1033, and the second sealing lid 1021b is returned to an original position by a restoring force of the second spring 1023b.

The first ventilation portion 102a, the connecting portion 103, and the second ventilation portion 102b can be sealed by such a sliding capping mechanism using the first sealing lid 1021a, the sealing lid 1031, and the second sealing lid 1021b. Furthermore, even in a case where tolerances occur due to manufacturing errors or variations in dimensions related to sealing, each of the first ventilation portion 102a, the connecting portion 103, and the second ventilation portion 102b can be sealed.

Note that, in the present embodiment, the mechanism may open and close the first sealing lid 1021a, the sealing lid 1031, and the second sealing lid 1021b in this order with a time difference in order to increase a releasing force of the odor component-containing air from the release ventilation opening 30b, or the mechanism may simultaneously open and close the first sealing lid 1021a, the sealing lid 1031, and the second sealing lid 1021b in order to enhance responsiveness.

Note that, in the opening and closing mechanism illustrated in Fig. 11, a coil spring is used as an example of the first spring 1023a, the spring 1033, and the second spring 1023b, but the present technology is not limited thereto, and an elastic body such as a leaf spring may be used. Furthermore, the first spring 1023a, the spring 1033, and the second spring 1023b may be the same elastic body or different elastic bodies.

### (7) Manufacturing example of cartridge 10 of first embodiment

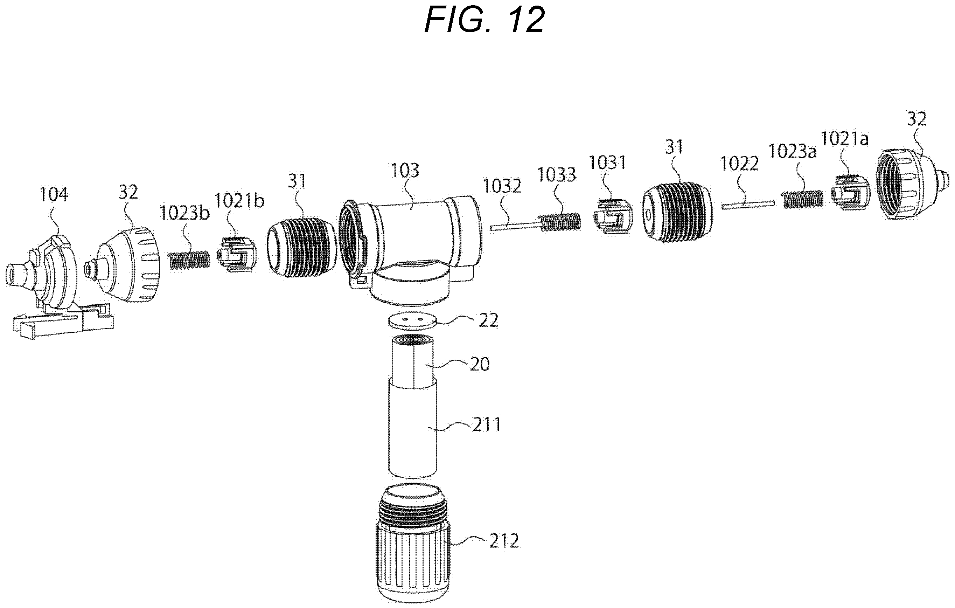

Fig. 12 is a perspective conceptual view illustrating a state in which the cartridge 10 is manufactured. Hereinafter, a manufacturing example of the cartridge 10 will be described with reference to Fig. 12.

As illustrated in Fig. 12, the cartridge 10 can be manufactured by engaging individual components constituting the cartridge 10 and confirming sealing property and the like. In engaging individual components, a component for enhancing sealability such as an O-ring may be used as necessary.

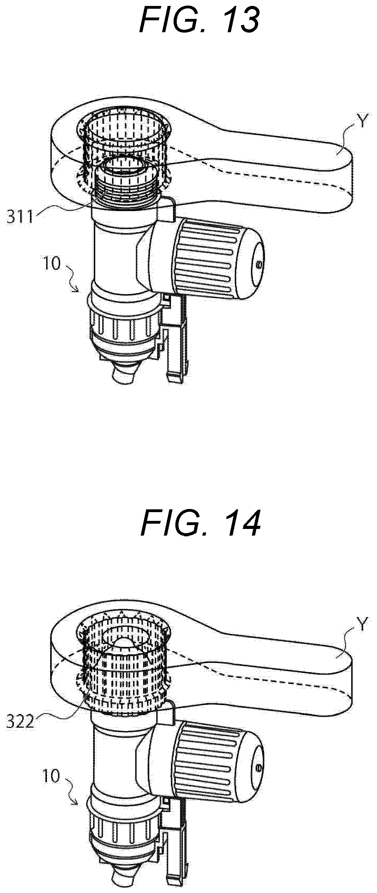

Furthermore, in engaging individual components, for example, as illustrated in Figs. 13 to 15, a dedicated jig Y having a groove or the like may be used. In this case, as illustrated in Fig. 13, the dedicated jig Y is preferably fitted to a part of the second fitting portion 311 provided in the inner layer member 31. As a result, tightening using the dedicated jig Y is facilitated, and the cartridge 10 with further improved sealability can be manufactured.

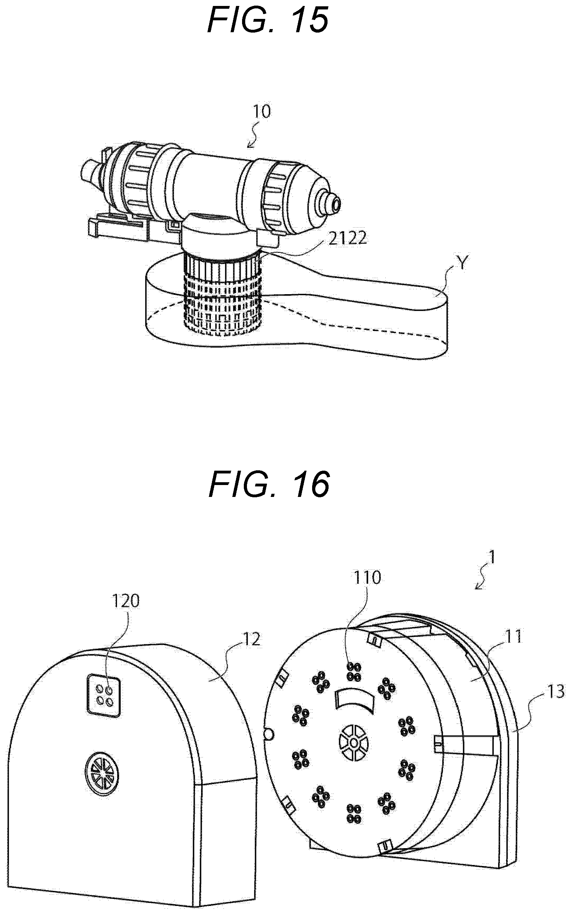

Furthermore, in the present embodiment, as illustrated in Figs. 14 and 15, a third fitting portion (322, 2122) for fitting with the dedicated jig Y may be provided in each of a part of the outer layer portion 212 and the outer layer member 32. As a result, tightening using the dedicated jig Y is facilitated, and the cartridge 10 with further improved sealability can be manufactured.

### 2. Second Embodiment (Cartridge 10 for Odor Generation Device)

### (1) Overall configuration

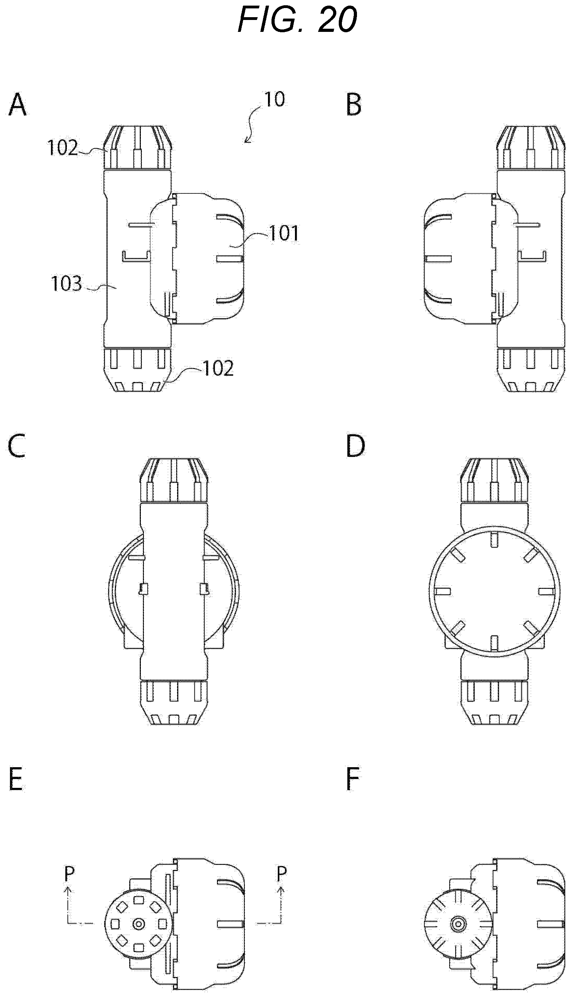

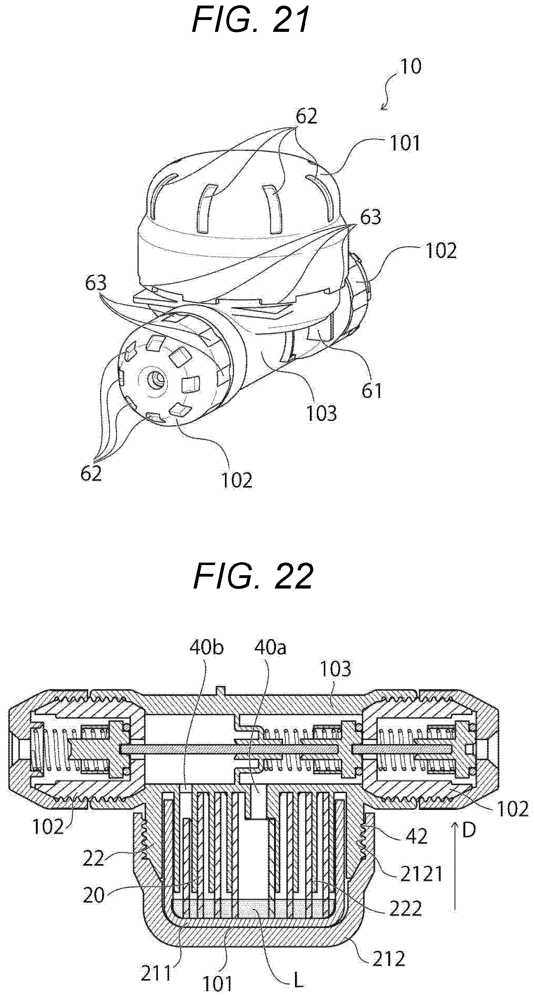

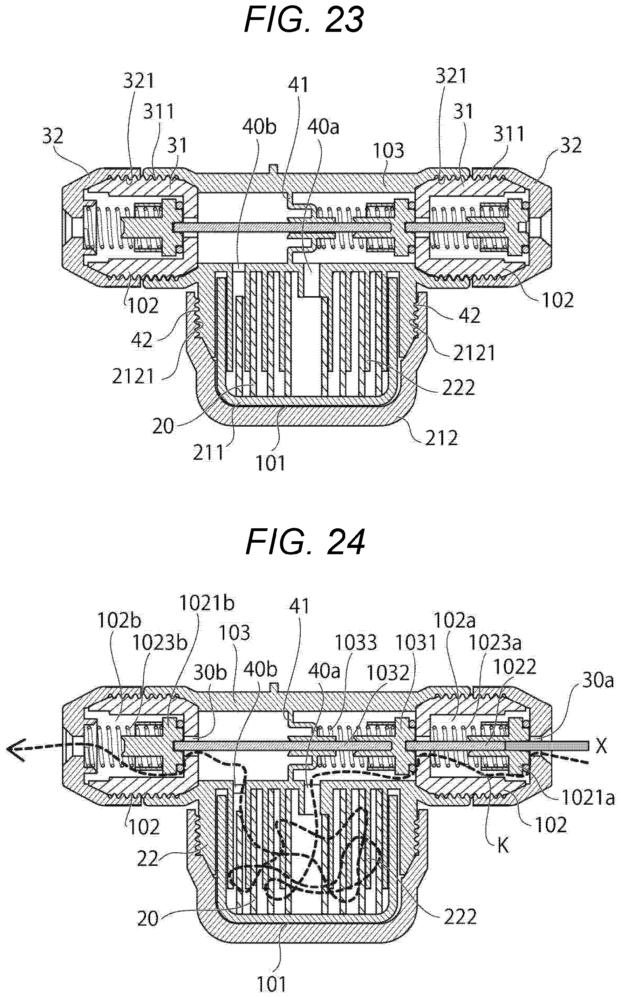

Fig. 20 is six views illustrating an embodiment of a cartridge 10 for an odor generation device (hereinafter, also referred to as a "cartridge 10") according to a second embodiment, in which A is a front view, B is a rear view, C is a left side view, D is a right side view, E is a plan view, and F is a bottom view. Furthermore, Fig. 21 is a perspective view illustrating an embodiment of the cartridge 10 for an odor generation device according to the second embodiment, and Figs. 22 and 23 are cross-sectional views taken along line P-P in the embodiment illustrated in Fig. 20.

The cartridge 10 includes at least an odor retaining portion 101, a ventilation portion 102, and a connecting portion 103. Adopting the cartridge 10 of the second embodiment makes it possible to keep in a part of an odor component including a liquid fragrance or the like, and handling becomes easy. For example, even if the cartridge 10 is disposed against gravity by using tension or capillary phenomenon, it is possible to retain the odor component, and conversely, it is possible to prevent the odor component from entering a portion that is not desired to enter. Furthermore, a structure is symmetrical, and adopting such a structure makes it possible to share components to be formed, and cost reduction can be achieved.

Hereinafter, details of each portion of the cartridge 10 of the second embodiment will be described.

### (2) Odor retaining portion 101

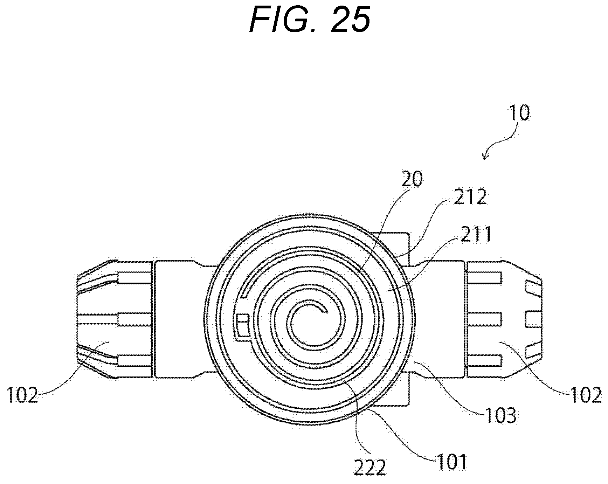

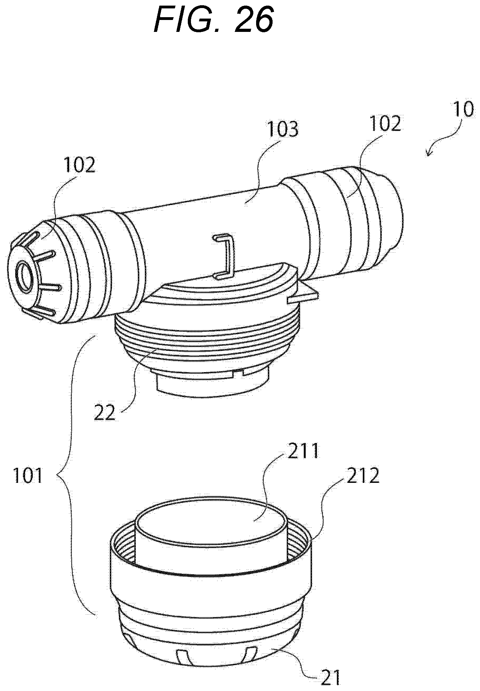

Fig. 25 is a view illustrating a state in which an outer layer portion 212 is removed and the odor retaining portion 101 is viewed from below (in the case of Fig. 26). Fig. 26 is a view illustrating a state in which a lid 22 of the odor retaining portion 101 is removed. The cartridge 10 includes the odor retaining portion 101 configured to retain an odor and the odor retaining portion 101 includes at least an impregnant 20 and a container portion 21, in the present embodiment. Furthermore, in the second embodiment, the lid 22 is joined to a part of the connecting portion 103 described later. Note that, in the present embodiment, the joining also includes an aspect in which the connecting portion 103 and the lid 22 are formed as a single molded component.

A material forming the impregnant 20 is not particularly limited as long as an odor component can be retained, and the material described in the first embodiment and the like can be used. Furthermore, an odor component retained in the impregnant 20 is not particularly limited.

The container portion 21 preferably has a two-layer structure, and includes an inner layer portion 211 forming an inner side for holding the impregnant 20 and the outer layer portion 212 forming an outer side of the container portion 21, in the present embodiment.

A form of the impregnant 20 is not particularly limited, but at least a part thereof preferably has a spiral structure. Specifically, for example, the sheetshaped impregnant 20 is preferably disposed in a spiral shape as illustrated in Fig. 25. As a result, an amount of the odor component impregnated in the impregnant 20 can be increased, and odor component-containing air does not reach a connection opening 40 unless the odor component-containing air always passes through a spiral side surface, so that an airflow containing a sufficient odor component can be generated. Furthermore, in this case, in order to maintain the spiral shape, as illustrated in Figs. 22 and 25, a rib 222 having a spiral shape or the like may be provided from an upper surface to a lower surface of the lid 22 (in the case of Fig. 26).

L of Fig. 22 indicates an odor component. A length of the impregnant 20 in a direction (direction of Arrow D in Fig. 22) orthogonal to the connecting portion 103 is preferably longer than a length of the inner layer portion 211 in the direction (direction of Arrow D in Fig. 22) orthogonal to the connecting portion 103. As a result, the odor component easily permeates the impregnant 20 (in particular, filter paper). Furthermore, in a case where the impregnant 20 contains a material softer than the inner layer portion 211, generation of a gap due to manufacturing variation can be absorbed by bending. Furthermore, a remaining space other than the inner layer portion 211 and the impregnant 20 can be reliably secured, and blocking of a flow of air can be prevented.

As a material for forming the inner layer portion 211 and the outer layer portion 212, the material described in the first embodiment and the like can be used.

A form of the inner layer portion 211 is not particularly limited as long as an odor component can be retained, and the form described in the first embodiment and the like can be used. However, as illustrated in Fig. 25, for example, a substantially cylindrical shape having an opening on an upper surface can be adopted. Furthermore, in this case, a form of the outer layer portion 212 can be a substantially cylindrical shape having an opening on an upper surface and having a diameter larger than that of the inner layer portion 211.

The outer layer portion 212 preferably includes a first fitting portion 2121 to be fitted to a first fitted portion 42 of the lid 22 joined to the connecting portion 103 described later. As a result, a sealing property can be improved. As a form of the first fitting portion 2121, the form described in the first embodiment and the like can be used.

### (3) Ventilation portion 102

Since a structure of the ventilation portion 102 is similar to that of the first embodiment, a description thereof is omitted here. However, a hole diameter of an inflow ventilation opening 30a of two ventilation openings 30 (30a, 30b) is preferably smaller than a hole diameter of a release ventilation opening 30b. In this way, odor component-containing air can be efficiently delivered to a user, and the user can more easily feel the odor.

### (4) Connecting portion 103

The connecting portion 103 includes: at least two connection openings 40 (40a, 40b) that are formed in the lid 22 joined to the connecting portion 103 and connect the ventilation portion 102 and the odor retaining portion 101; and a partition 41 disposed upstream of one connection opening among the at least two connection openings 40 (40a, 40b).

In the present embodiment, the two connection openings 40 (40a, 40b) preferably include: a first connection opening 40a configured to release air from the connecting portion 103 to the odor retaining portion 101; and a second connection opening 40b configured to release odor component-containing air from the odor retaining portion 101 to the connecting portion 103. In this case, the air having flowed into the connecting portion 103 via a first ventilation portion 102a is released to the odor retaining portion 101 via the first connection opening 40a and is mixed with the odor component, and thereafter, the odor component-containing air flows into the connecting portion 103 via the second connection opening 40b and is released to the outside via a second ventilation portion 102b.

In the present embodiment, the partition 41 is disposed upstream of the second connection opening 40b among the two connection openings 40 (40a, 40b). By including the partition 41, air having flowed into the connecting portion 103 forcibly becomes odor component-containing air via the odor retaining portion 101, and flows into the connecting portion 103 again. As a result, odor component-containing air can be efficiently generated.

Furthermore, in the present embodiment, the connecting portion 103 may include an opening and closing mechanism in a connection region between with the ventilation portion 102. As the opening and closing mechanism, the opening and closing mechanism described in the first embodiment or the like can be used. In this case, while a shaft 1032 performs an operation of pushing from the upstream side of the cartridge 10, the partition 41 is preferably configured not to hinder the operation of the shaft 1032 as illustrated in Fig. 23.

As illustrated in Fig. 23, the lid 22 joined to the connecting portion 103 preferably includes the first fitted portion 42 to be fitted to the first fitting portion 2121 described above. As a result, a sealing property can be improved. Note that, in a case where the form of the first fitting portion 2121 is a protruding screw formed in the fitting region, the first fitted portion 42 is preferably a recessed screw.

As a material for forming the lid 22, the material described in the first embodiment and the like can be used.

As a form of the lid 22, a shape that is easy to fit with the above-described outer layer portion 212 is preferable, and for example, it is possible to adopt a shape having an opening on a bottom surface (in the case of Fig. 26), an upper surface joined to the connecting portion 103, and the above-described first fitted portion 42 on at least a part of a side surface.

### (5) Operation example of cartridge 10 of second embodiment

Fig. 24 is a conceptual cross-sectional view illustrating a state in which an odor is released from the cartridge 10 of the second embodiment. Hereinafter, with reference to Fig. 24, an operation example of releasing odor component-containing air in the cartridge 10 will be described.

In the present embodiment, when the ventilation portion 102 is opened in a state where an odor component is retained in the odor retaining portion 101, air flows in from the outside. The air having flowed in flows into the connecting portion 103, is mixed with the odor component through the one connection opening 40a, and is released as odor component-containing air. In this state, the released odor component-containing air flows into the connecting portion 103 via another connection opening 40b, and is thereby released to the outside.

Specifically, in the present embodiment, the ventilation opening 30 (30a, 30b) is provided with an opening and closing mechanism including a sealing lid 1021 (1021a, 1021b), a shaft 1022, and a spring 1023 (1023a, 1023b), and the connecting portion 103 is also provided with an opening and closing mechanism including a sealing lid 1031, the shaft 1032, and a spring 1033. These opening and closing mechanisms can be controlled, for example, by inserting a pusher X from an odor generation device 1 side or the like, from a lower portion of the cartridge 10.

When the pusher X is pressed, the pusher X pushes a first sealing lid 1021a in an inward direction of the first ventilation portion 102a, the inflow ventilation opening 30a is opened, and air flows into the first ventilation portion 102a. At this time, a first spring 1023a is contracted by the first sealing lid 1021a.

When the first sealing lid 1021a is pushed in the inward direction of the first ventilation portion 102a, the shaft 1022 mounted to the first sealing lid 1021a moves in a direction of the connecting portion 103. The shaft 1022 pushes the sealing lid 1031 in an inward direction of the connecting portion 103, and air in the first ventilation portion 102a flows into the connecting portion 103. At this time, the spring 1033 is contracted by the sealing lid 1031.

Since there is the partition 41, the air having flowed into the connecting portion 103 first flows into the odor retaining portion 101 through the first connection opening 40a provided in the lid 22 and is mixed with the odor component retained in the odor retaining portion 101, and odor component-containing air is generated. The odor component-containing air flows into the connecting portion 103 again through the second connection opening 40b provided in the lid 22.

When the sealing lid 1031 is pushed in the inward direction of the connecting portion 103, the shaft 1032 mounted to the sealing lid 1031 moves in a direction of the second ventilation portion 102b. The shaft 1032 pushes a second sealing lid 1021b in an inward direction of the second ventilation portion 102b, and the odor component-containing air in the connecting portion 103 flows into the second ventilation portion 102b. At this time, a second spring 1023b is contracted by the second sealing lid 1021b.

In the present embodiment, since the second ventilation portion 102b is connected to the release ventilation opening 30b, the odor component-containing air having flowed into the second ventilation portion 102b is released to the outside from the release ventilation opening 30b.

When the pressing to the pusher X is released after the odor component-containing air is released to the outside, the first sealing lid 1021a is returned to an original position by a restoring force of the contracted first spring 1023a. Furthermore, the sealing lid 1031 is returned to an original position by a restoring force of the spring 1033, and the second sealing lid 1021b is returned to an original position by a restoring force of the second spring 1023b.

### (6) Manufacturing example of cartridge 10 of second embodiment

Similarly to the cartridge 10 of the first embodiment, the cartridge 10 of the second embodiment can be manufactured by sequentially fastening individual portions by using a dedicated jig, a torque wrench, or the like.

In the present embodiment, as illustrated in Fig. 21, at least a part of an outer periphery of the connecting portion 103 preferably has a protrusion 61 that prevents reverse assembly. As a result, productivity of the cartridge 10 can be improved.

Furthermore, in the present embodiment, as illustrated in Fig. 21, knurling 62 is preferably applied to at least a part of an outer periphery of the outer layer portion 212 and/or an outer layer member 32. As a result, it is possible to further strengthen the engagement of individual portions at the time of fastening, and to improve sealability of the cartridge 10.

Moreover, in the present embodiment, as illustrated in Fig. 21, a hooking portion 63 is preferably provided on at least a part of an outer periphery of the outer layer portion 212 and/or the outer layer member 32 to avoid failure when taken out of a mold. As a result, it is possible to smoothly take out the cartridge 10 from the mold and to provide the cartridge 10 suitable for mass production.

### 3. Third Embodiment (Cartridge 10 for Odor Generation Device)

### (1) Overall configuration

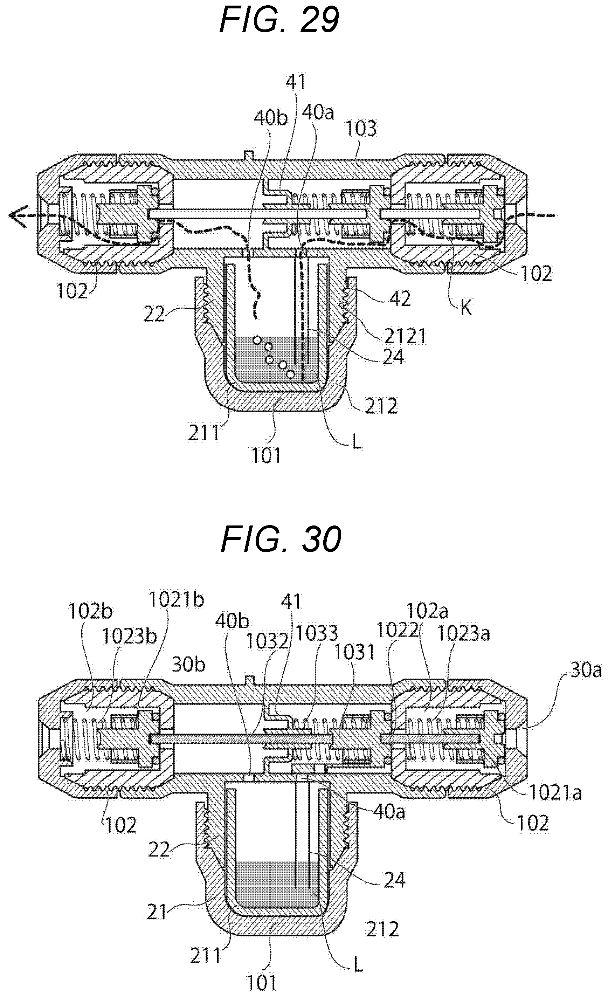

Fig. 29 is a cross-sectional view illustrating an embodiment of a cartridge 10 for an odor generation device (hereinafter, also referred to as a "cartridge 10") according to a third embodiment.

The cartridge 10 includes at least an odor retaining portion 101, a ventilation portion 102, and a connecting portion 103. Adopting the cartridge 10 of the third embodiment makes it possible to more uniformly release odor component-containing air. For example, it is possible to uniformly provide odor component-containing air as compared with the cartridge 10 having a structure in which a retention amount of an odor component changes due to a positional change or a temporal change. Furthermore, there is also an advantage that a mechanism (for example, the fixing portion 23, the rib 222, and the like) for holding the impregnant 20 becomes unnecessary and a degree of freedom of a structure increases. Furthermore, similarly to the cartridge 10 of the third embodiment, a structure is symmetrical, and adopting such a structure makes it possible to share components to be formed, and cost reduction can be achieved.

Hereinafter, details of each portion of the cartridge 10 of the third embodiment will be described.

### (2) Odor retaining portion 101

The cartridge 10 includes the odor retaining portion 101 configured to retain an odor and the odor retaining portion 101 includes at least a container portion 21 and a tubular body 24 in the present embodiment. Note that, as illustrated in Fig. 29, the tubular body 24 is preferably disposed on the upstream side of a partition 41 and connected to a connection opening 40a. Furthermore, the tubular body 24 may be a single molded part with the lid 22, or may be a separate part.

Furthermore, in the third embodiment, the lid 22 is joined to a part of the connecting portion 103 similarly to the second embodiment. Also in the present embodiment, the joining includes an aspect in which the connecting portion 103 and the lid 22 are formed as a single molded component. Furthermore, although not illustrated in Fig. 29, an impregnant 20, a fixing portion 23, and the like may be provided in a part of the container portion 21 as necessary.

The container portion 21 preferably has a two-layer structure. As a material for forming an inner layer portion 211 and an outer layer portion 212, the material described in the first embodiment and the like can be used.

In the present embodiment, the tubular body 24 is provided from the lid 22 side toward a bottom portion of the inner layer portion 211. For example, in a case where the inner layer portion 211 retains a liquid fragrance or the like as an odor component, when an airflow is sent from the tubular body 24, the airflow is imparted with scent when passing through the liquid, and the airflow flows in an outlet direction as it is, thereby odor component-containing air can be generated from a release ventilation opening 30b of the cartridge 10.

A length of the tubular body 24 is not particularly limited, but is preferably from below a liquid level of the liquid fragrance to above the bottom portion of the inner layer portion 211. Furthermore, examples of a material forming the tubular body 24 include, but not limited to, Teflon (registered trademark), a fluorine tube, a silicon tube, a urethane tube, and the like.

A form of the inner layer portion 211 is not particularly limited as long as an odor component can be retained, and the form described in the second embodiment and the like can be used. However, the inner layer portion preferably contains a material that does not infiltrate the liquid fragrance and the like and is less likely to cause chemical changes such as oxidation. Specific examples thereof include aluminum (that may have an oxide film), glass, and the like, but not limited thereto. Furthermore, in this case, a form of the outer layer portion 212 can be a substantially cylindrical shape having an opening on an upper surface and having a diameter larger than that of the inner layer portion 211. Furthermore, the outer layer portion 212 may include a first fitting portion 2121 to be fitted to a first fitted portion 42 of the lid 22 joined to the connecting portion 103 described later.

### (3) Ventilation portion 102

Since a structure of the ventilation portion 102 is similar to that of the first embodiment, a description thereof is omitted here.

### (4) Connecting portion 103

Since a configuration of the connecting portion 103 is the same as that of the second embodiment, a description thereof will be omitted here. However, as a material for forming the lid 22, at least the inside thereof is preferably a material that does not infiltrate the liquid fragrance or the like and is less likely to cause chemical changes such as oxidation, similarly to the inner layer portion 211. For example, in a case where the lid 22 is formed containing resin, it is conceivable that the inside thereof is made containing aluminum, glass, or the like. As a result, in a case where the odor component is a liquid fragrance or the like, it is possible to prevent the liquid from directly touching the resin. Furthermore, a packing (for example, a fluorine-based rubber packing or the like) may be provided between the lid 22 and the inner layer portion 211 described above in order to improve sealability.

Furthermore, a sealing lid 1031 constituting an opening and closing mechanism in the connecting portion 103 may have a tubular body opening and closing mechanism that opens and closes an upper surface (that is, the lid 22 side) at a position where the tubular body 24 in the odor retaining portion 101 reaches the lid 22.

### (5) Operation example of cartridge 10 of third embodiment

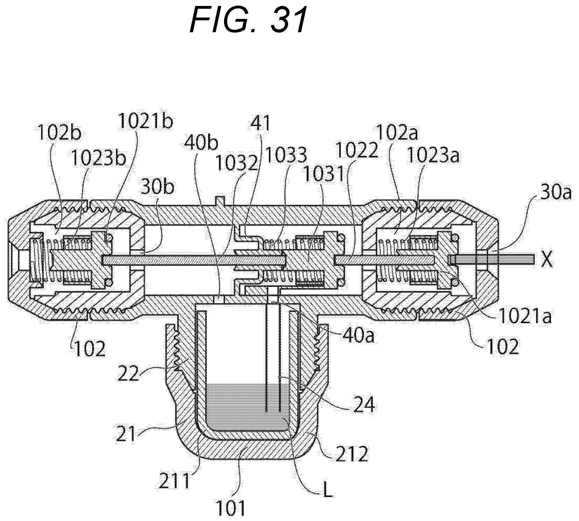

Figs. 30 and 31 are conceptual cross-sectional views illustrating a state in which an odor is released from the cartridge 10 of the third embodiment. Hereinafter, with reference to Figs. 30 and 31, an operation example of releasing odor component-containing air in the cartridge 10 will be described. Note that an odor component L here is assumed to be a liquid fragrance or the like.

In the present embodiment, when the ventilation portion 102 is opened in a state where the odor component such as the liquid fragrance is retained in the odor retaining portion 101, air flows in from the outside. The air having flowed in flows into the connecting portion 103, passes through the tubular body 24 through one connection opening 40a, and sends an airflow to the liquid odor component. The airflow is imparted with scent when passing through the liquid, and odor component-containing air is released. In this state, the released odor component-containing air flows into the connecting portion 103 via another connection opening 40b, and is thereby released to the outside.

Specifically, in the present embodiment, a ventilation opening 30 (30a, 30b) is provided with an opening and closing mechanism including a sealing lid 1021 (1021a, 1021b), a shaft 1022, and a spring 1023 (1023a, 1023b), and the connecting portion 103 is also provided with an opening and closing mechanism including the sealing lid 1031 with a tubular body opening and closing mechanism, a shaft 1032, and a spring 1033. These opening and closing mechanisms can be controlled, for example, by inserting a pusher X from the odor generation device 1 side or the like, from a lower portion of the cartridge 10.

When the pusher X is pressed, the pusher X pushes a first sealing lid 1021a in an inward direction of a first ventilation portion 102a, an inflow ventilation opening 30a is opened, and air flows into the first ventilation portion 102a. At this time, a first spring 1023a is contracted by the first sealing lid 1021a.

When the first sealing lid 1021a is pushed in the inward direction of the first ventilation portion 102a, the shaft 1022 mounted to the first sealing lid 1021a moves in a direction of the connecting portion 103. The shaft 1022 pushes the sealing lid 1031 with the tubular body opening and closing mechanism in an inward direction of the connecting portion 103, and air in the first ventilation portion 102a flows into the connecting portion 103. At this time, the spring 1033 is contracted by the sealing lid 1031 with the tubular body opening and closing mechanism. Here, a mechanism is adopted in which the sealing lid 1031 with the tubular body opening and closing mechanism in the state of Fig. 30 before being pushed by the pusher X is brought into the state of Fig. 31 by being pushed by the pusher X, that is, by being pushed in an inward direction of the cartridge 10, and an upper surface of the tubular body 24 is opened.

Since there is the partition 41, the air having flowed into the connecting portion 103 first passes through the tubular body 24 in an opened state through a first connection opening 40a provided in the lid 22, flows into the odor retaining portion 101, and sends an airflow to the liquid odor component retained by the odor retaining portion 101. As a result, bubbling occurs, the airflow is imparted with scent when passing through the liquid, and the odor component-containing air is released. The odor component-containing air flows into the connecting portion 103 again through a second connection opening 40b provided in the lid 22.

When the sealing lid 1031 with the tubular body opening and closing mechanism is pushed in the inward direction of the connecting portion 103, the shaft 1032 mounted to the sealing lid 1031 with the tubular body opening and closing mechanism moves in a direction of a second ventilation portion 102b. The shaft 1032 pushes a second sealing lid 1021b in an inward direction of the second ventilation portion 102b, and the odor component-containing air in the connecting portion 103 flows into the second ventilation portion 102b. At this time, a second spring 1023b is contracted by the second sealing lid 1021b.

In the present embodiment, since the second ventilation portion 102b is connected to the release ventilation opening 30b, the odor component-containing air having flowed into the second ventilation portion 102b is released to the outside from the release ventilation opening 30b.

When the pressing to the pusher X is released after the odor component-containing air is released to the outside, the first sealing lid 1021a is returned to an original position by a restoring force of the contracted first spring 1023a. Furthermore, the sealing lid 1031 with the tubular body opening and closing mechanism is returned to an original position by a restoring force of the spring 1033, and the second sealing lid 1021b is returned to an original position by a restoring force of the second spring 1023b.

### (6) Manufacturing example of cartridge 10 of third embodiment

Since a method of manufacturing the cartridge 10 is similar to that of the second embodiment, a description thereof is omitted here.

### 4. Fourth Embodiment (Odor Generation Device 1)

### (1) Overall configuration

Fig. 16 is an exploded perspective view illustrating an embodiment of an odor generation device 1 according to a fourth embodiment. The odor generation device 1 according to the present technology includes the cartridge 10 of the first embodiment described above and a cartridge holding unit 11. Furthermore, a front storage portion 12, a back storage portion 13, and the like may be provided as necessary. Note that, since the cartridge 10 of the first embodiment is as described above, a description thereof will be omitted here.

Hereinafter, details of each unit of the odor generation device 1 according to the fourth embodiment will be described.

### (2) Cartridge holding unit 11

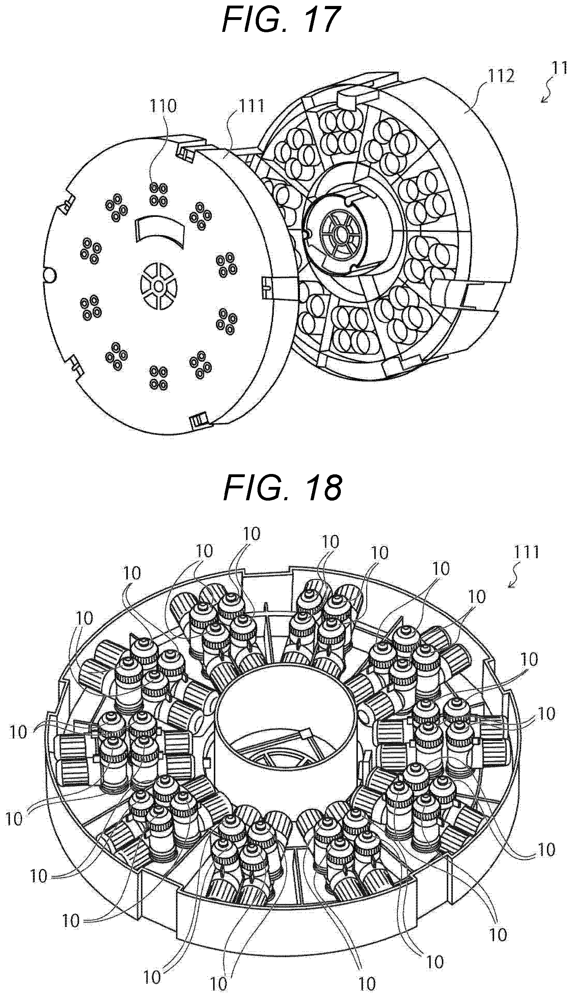

Fig. 17 is an exploded perspective view illustrating an embodiment of the cartridge holding unit 11, and Fig. 18 is a perspective view illustrating an embodiment of an indwelling portion 111 constituting the cartridge holding unit 11. The cartridge holding unit 11 holds one or two or more cartridges 10. Specifically, as illustrated in Figs. 17 and 18, the cartridge holding unit 11 includes: the indwelling portion 111 allowing indwelling of one or more cartridges 10 and having a discharge hole 110 for discharge of odor component-containing air released from the cartridge 10; and a holding portion 112 to be fitted to the indwelling portion 111 to hold the cartridge 10.

The number of cartridges 10 to be held is not particularly limited, and can be freely set according to a purpose of use or the like of the odor generation device 1. For example, in a case of using the odor generation device 1 for a reference olfactory examination in Japan, the number may be 40 (five kinds of olfactory elements ×5 are set as the highest concentrations, and the concentrations are set to eight stages of 5, 4, 3, 2, 1, 0, -1, and -2, sequentially in units of 10 times. Note that only one type of olfactory element among the five types of olfactory elements has no level 5, and has seven stages). Other than Japan, the cartridge 10 may be held such that the number of odors according to an embodiment of the country can be generated. Furthermore, in a case where the purpose of use of the odor generation device 1 is relaxation or the like, a structure of the cartridge holding unit 11 can be designed so that a desired number of cartridges 10 can be held.

In the present embodiment, the holding portion 112 that holds the plurality of cartridges 10 at a time as described above may be handled as a unit-type cartridge holding unit 11 (that is, a cartridge unit and the like), and attachment, replacement, and distribution to the generation device 1 may be performed on a unit basis.

A form of the indwelling portion 111 and/or the holding portion 112 is not particularly limited, and can be freely designed according to a form of the cartridge 10 to be held and the like. For example, the indwelling portion 111 and/or the holding portion 112 can be formed in a substantially rectangular parallelepiped shape, a substantially cylindrical shape, a substantially cubic shape, or the like.

A material forming the indwelling portion 111 and/or the holding portion 112 is not particularly limited as long as the cartridge 10 can be held. Specifically, the indwelling portion 111 and/or the holding portion 112 contains, for example, an organic polymer material. As the organic polymer material, for example, it is possible to use polyvinyl chloride, polyethylene, a phenol resin, an olefin resin, nylon, polyester, a synthetic rubber, a silicone resin, a natural rubber, a protein, a nucleic acid, a lipid, polysaccharide, or the like, or a material obtained by combining two or more of them. Furthermore, in addition to this, for example, it is also possible to use a polymer resin such as an acrylic resin, a urethane resin, an ABS resin, a polyether ether ketone (PEEK) resin, a polyacetal (POM) resin, a fluororesin, a cycloolefin polymer resin, or a polyimide resin, a metal such as stainless steel or aluminum, an inorganic crystal such as quartz, glass, or the like, or a material obtained by combining two or more of them.

Furthermore, the indwelling portion 111 and/or the holding portion 112 may be formed porous, and for example, a mesh structure, cork, mesoporous silica, calcium carbonate, or the like can be used. Note that the indwelling portion 111 and/or the holding portion 112 may have a fiber structure or a layered structure (for example, clay minerals and the like) other than the porous structure. Furthermore, the indwelling portion 111 and/or the holding portion 112 may be formed using ceramic or the like.

### (3) Front storage portion 12

In the front storage portion 12, a release hole 120 is a portion for releasing odor component-containing air to the outside. For example, as illustrated in Fig. 16, the release hole 120 may be provided in a part of the front storage portion 12, and in this case, may be connected to the discharge hole 110 described above.

Furthermore, the front storage portion 12 may include a guide unit (not illustrated) that guides odor component-containing air in the vicinity of the nose of the user. The material forming the guide unit is not particularly limited, and examples thereof include paper (including recycled paper), wood, bamboo sheath, plastic, coal, and the like. A part or all of the guide unit may be detachably formed, and in this case, for example, may be disposable for each user.

### (4) Back storage portion 13

The back storage portion 13 includes a drive mechanism unit. The drive mechanism unit includes a drive mechanism accommodating portion, and is connected to an operation shaft and a shaft 1022 in the cartridge 10 to drive these. The drive mechanism accommodating portion can have, for example, a cylindrical shape, but can be formed in a substantially cylindrical shape, a substantially rectangular parallelepiped shape, a substantially cubic shape, or the like in accordance with a shape of the cartridge 10.

The drive mechanism unit includes a pusher connected to the operation shaft and a shape memory alloy SMA of a thin wire which is a drive source for driving the pusher inside the drive mechanism accommodating portion. A rear end of the pusher is fixed to a drive mechanism fixing portion provided at the inner rear end of the drive mechanism accommodating portion. An SMA sliding portion for folding back and sliding the shape memory alloy SMA is provided near the distal end of the pusher. Furthermore, the entire drive mechanism unit is fixed by a support or the like mounted below the drive mechanism accommodating portion, and a wiring capable of supplying power is connected to a rear end of the shape memory alloy SMA located in the drive mechanism fixing portion.

The pusher is movable inside the drive mechanism accommodating portion in the extending direction by expansion and contraction of the shape memory alloy SMA. The shape of the pusher is not particularly limited as long as it is a shape that pushes the operation shaft, and may be a cylindrical shape, a conical shape, a cylindrical shape, a prismatic shape, or the like.

The shape memory alloy SMA is folded back in a U-shape at the SMA sliding portion provided near the distal end of the pusher and passes through the inside of the pusher, and both ends thereof are fixed to the drive mechanism fixing portion located at the rear end of the pusher. Moreover, the actuator as the drive source is not limited to the shape memory alloy SMA, and may be, for example, a linear motion mechanism that linearly moves a pusher such as a motor, a solenoid, a linear slide type, a pneumatic (air pump type), or a small electromagnet. Here, the linear motion mechanism includes not only a case where one member moves in the linear direction but also a case where a part of a plurality of members connected to each other moves in the linear direction.

### (5) Operation example of odor generation device 1 of fourth embodiment

Hereinafter, an operation example of generating odor component-containing air from the odor generation device 1 will be described.

First, the cartridge 10 is attached to the odor generation device 1. In a case where the number of the cartridges 10 is small, it is also possible to attach one each to a predetermined place. In a case where the number of cartridges 10 to be attached is large, a plurality of cartridges 10 can be attached to the odor generation device 1 at a time by using a unit-type cartridge holding unit 11 (that is, a cartridge unit) including the holding portion 112 that holds the plurality of cartridges 10 in advance as illustrated in Fig. 18.

Next, for example, in a case where a plurality of cartridges 10 is attached to the odor generation device 1, when an instruction to generate the odor component-containing air is received from the outside, the pusher is moved in a direction of the cartridge 10 by the drive mechanism unit connected to the cartridge 10. When the operation shaft connected to the pusher moves in the direction of the cartridge 10, an inflow ventilation opening 30a opens, and air flows into a first ventilation portion 102a. When air flows into the cartridge 10, odor component-containing air is generated in the cartridge 10. Since the generation of odor component-containing air is as described above, a description thereof is omitted here.

Then, the odor component-containing air released from a releasing portion 104 of the cartridge 10 is released from the release hole 120 via a discharge hole 110 of the cartridge holding unit 11, and guided to the vicinity of the nose of the user.

### (6) Usage example of odor generation device 1 of fourth embodiment

The odor generation device 1 is used, for example, as a device that releases an odor to a space in a limited range, and is specifically used for an olfactory examination, olfactory training (olfactory stimulation therapy), and the like. Note that the olfactory training referred to herein is interpreted in a broad sense, and can include practice of an odor determination test, a sommelier test, an aromatherapy verification, and the like. Moreover, the odor generation device 1 may be used for flavor simulation in development of food and drink. Furthermore, the film may be mounted on an automobile, a head mounted display, a relaxed product such as a neck pillow or an eye pillow, or the like. In the case of being mounted on an automobile, for example, an odor can be generated on the basis of an instruction of a driver or a passenger, position information of the automobile, movement of the driver or the passenger, a biological signal, or the like may be detected, and the odor may be generated on the basis of the detection result. In the case of being mounted on a head mounted display, for example, an odor can be generated in conjunction with an image presented on the display, a motion of the user, a biological signal, or the like may be detected, and the odor may be generated on the basis of the detection result. In the case of being mounted on a relaxation product such as a neck pillow or an eye pillow, for example, an odor can be generated on the basis of an instruction of a user, a motion of the user or a biological signal may be detected, and the odor may be generated on the basis of the detection result.

Furthermore, the odor generation device 1 may be used as a device that releases an odor to a wide range of space, and specifically, the odor generation device 1 can be installed on a vending machine, a digital signage, or a customer attracting product such as a robot. In the case of being mounted on a customer attracting product, for example, actions, facial expressions, and the like of an unspecified number of users can be detected, and an odor can be generated on the basis of the detection result.

The odor generation device 1 may be a portable device that can be carried by a user or may be a stationary device.

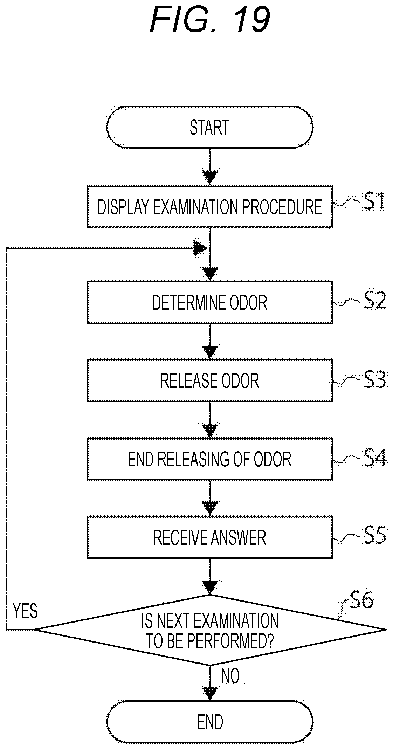

Fig. 19 is a flowchart for explaining an operation example of an olfactory examination or olfactory training. Hereinafter, with reference to Fig. 19, a case where the odor generation device 1 is used as an olfactory examination device will be described.

First, an examiner such as a doctor, a nurse, or a laboratory technician instructs an examinee to display an examination procedure using an information processing device or the like (S1). Note that, in the present embodiment, various devices having a user interface unit may be provided not only on the examiner side but also on the examinee side. Next, the examiner determines an odor (olfactory element) to be released to the examinee, and instructs the odor generation device 1. At this time, an arrangement drive unit having received the instruction arranges the cartridge 10 having a target odor component near the release hole 120, and releases odor component-containing air according to the operation example of the odor generation device 1 described above (S3).

Next, when the release of the odor component-containing air ends (S4), an answer from the examinee is received, and a storage unit or the like stores the answer (S5). Next, the examiner determines whether or not to perform the next examination (S6), and in the case of Yes, returns to step S2 again and determines the next odor. In a case where the arrangement drive unit is rotationally driven, at a time of placing the next specific cartridge 10 near the release hole 120, a deodorizing operation may be performed according to the operation example of the odor generation device 1 described above. In the case of No, the examination ends as it is.

### 5. Fifth Embodiment (Odor Generation Device 1)

### (1) Overall configuration

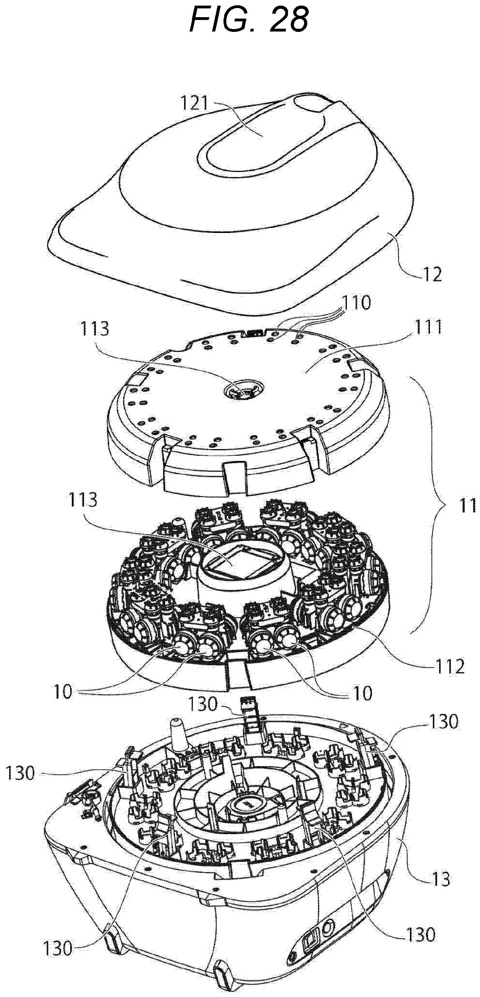

Fig. 28 is an exploded perspective view illustrating an embodiment of an odor generation device 1 according to a fifth embodiment. The odor generation device 1 according to the present technology includes the cartridge 10 of the second embodiment described above and a cartridge holding unit 11. Furthermore, a front storage portion 12, a back storage portion 13, and the like may be provided as necessary. Note that, since the cartridge 10 of the second embodiment is as described above, a description thereof will be omitted here.

Hereinafter, details of each unit of the odor generation device 1 according to the fifth embodiment will be described. Note that, in the odor generation device 1 of the fifth embodiment, the housing itself may have an inclination of several degrees so that the user can easily bring the nose close to a release hole 120.

### (2) Cartridge holding unit 11

The cartridge holding unit 11 holds one or two or more cartridges 10. Specifically, as illustrated in Fig. 28, the cartridge holding unit 11 includes: an indwelling portion 111 allowing indwelling of one or more cartridges 10 and having a discharge hole 110 for discharge of odor component-containing air released from the cartridge 10: and a holding portion 112 to be fitted to the indwelling portion 111 to hold the cartridge 10. Furthermore, a deodorization unit 113 containing a deodorizer and/or a deodorant may be provided as necessary.

The number of the cartridges 10 to be held is not particularly limited, and is similar to that of the fourth embodiment, and thus a description thereof is omitted here.

A form of the indwelling portion 111 and/or the holding portion 112 is not particularly limited, and can be freely designed according to a form of the cartridge 10 to be held and the like. For example, the indwelling portion 111 and/or the holding portion 112 can be formed in a substantially disk shape, a substantially rectangular parallelepiped shape, a substantially cylindrical shape, a substantially cubic shape, or the like.

A material for forming the indwelling portion 111 and/or the holding portion 112 is not particularly limited, and is similar to that of the fourth embodiment, and thus a description thereof is omitted here.

Moreover, the indwelling portion 111 and/or the holding portion 112 can contribute to the SDGs by being formed using recycled plastic or the like. Furthermore, by separately forming the indwelling portion 111 and the holding portion 112, only the holding portion 112 that holds the cartridge 10 is handled as medical waste, and the indwelling portion 111, the front storage portion 12, the back storage portion 13, and the like other than that can be reused without being discarded. As a result, it is possible to improve recyclability, reduce medical waste, and reduce the number of parts at the time of replacing the cartridge 10.

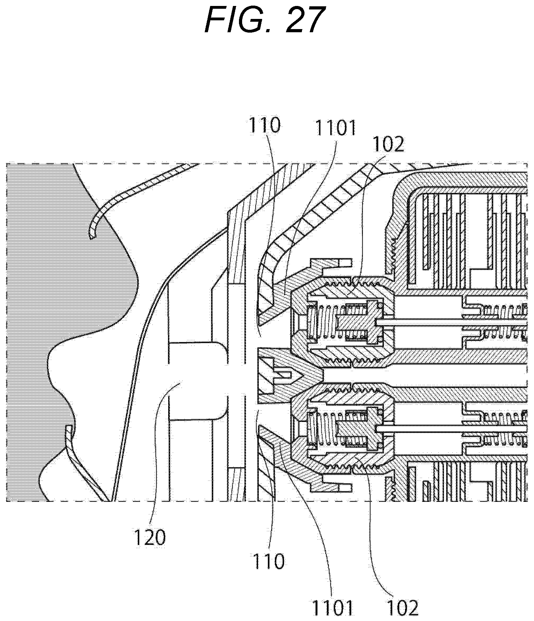

Fig. 27 is an enlarged view of a main part of a nozzle-shaped portion 1101 in the odor generation device 1 according to the fifth embodiment. In the present embodiment, in the cartridge holding unit 11, the indwelling portion 111 may include the nozzle-shaped portion 1101 that discharges an airflow to a desired position, on the cartridge 10 side. In this case, the cartridge 10 may not include the releasing portion 104 as in the first embodiment. By including the nozzle-shaped portion 1101, it is possible to discharge odor component-containing air toward a subnasal portion of the user, and it is possible to cause the effective user to inhale the odor component-containing air.

A surface of the nozzle-shaped portion 1101 is preferably a smooth surface with reduced roughness. As a result, the odor can be prevented from remaining in the nozzle-shaped portion 1101. Furthermore, a material of the nozzle-shaped portion 1101 is not particularly limited, but is preferably a material that is less likely to cause odor residue.

The deodorization unit 113 is a portion that deodorizes the remaining odor component-containing air staying in a guide unit 121. For example, as illustrated in Fig. 28, the deodorization unit 113 is disposed in a part of the indwelling portion 111 and the holding portion 112. The deodorization unit 113 may operate in a vertical direction, a horizontal direction, or a rotation direction, and a power source in that case may be an actuator or an airflow.

A form of the deodorization unit 113 is not particularly limited, but may be a filter structure. In this case, for example, a slit structure in which a gas adsorbing substance such as activated carbon is woven or kneaded, or a fiber or a structure itself has a gas adsorbing structure can be applied to the filter. Furthermore, as the slit structure, it is possible to adopt a linear slit, a honeycomb structure, a structure in which a substantially circular shape, a substantially square shape, or the like is divided by a plurality of straight lines such that each area is equally spaced, or the like.

### (3) Front storage portion 12

Similarly to the odor generation device 1 described in the fourth embodiment, the front storage portion 12 includes the release hole 120 through which odor component-containing air is discharged to the outside. For example, the release hole 120 may be provided in a part of the front storage portion 12, and in this case, may be connected to the discharge hole 110 described above.

Furthermore, the front storage portion 12 may include the guide unit 121 that guides odor component-containing air toward the nose of the user. The guide unit 121 may include, for example, a nose cover portion that covers the nose of the user, an opening provided on an upper end side of the nose cover portion, and a guiding portion that guides the remaining odor component-containing air to the deodorization unit 113. The guide unit 121 may be three-dimensionally formed in accordance with a shape of a mouth and a nose, for example, the guiding portion may have a recess and may have a structure in which the user's mouth can be easily accommodated. Furthermore, this makes it easy to store the guide units 121 in an overlapping manner, so that the flowability, the storage property, and the shape maintainability are also improved.

The guide unit 121 is a part necessary for deodorizing the discharged odor component-containing air, but is preferably disposable each time from the viewpoint of hygiene reasons, odor residue, and the like because a user such as a subject sticks his/her nose.

### (4) Back storage portion 13

The back storage portion 13 includes a drive mechanism unit and an arrangement drive unit. Since the drive mechanism unit is similar to that of the fourth embodiment, a description thereof is omitted here. Furthermore, a fitting portion 130 to be fitted to the holding portion 112 (or the cartridge unit or the like) may be provided as necessary.

The arrangement drive unit is a portion that arranges a specific cartridge 10 among the plurality of cartridges 10 in the vicinity of the discharge hole 110. Since the odor generation device 1 includes the arrangement drive unit, any cartridge 10 can be moved to the vicinity of the discharge hole 110, and an odor component retained by an odor retaining portion 101 of the cartridge 10 can be delivered to a desired position (for example, a tip of the nose of the user, or the like). Furthermore, as a result, it is possible to suppress as much as possible the odor from adhering to the members constituting the odor generation device 1.

The arrangement drive unit can be driven in conformity with the form of the holding portion 112, the cartridge unit, and the like, and can be driven to, for example, linear drive, XY axis drive, rotational drive, and the like. The arrangement drive unit is not particularly limited, and a conventionally known actuator or the like can be used.

A fitting portion 150 is preferably configured to be able to withstand a load applied in an attaching and detaching direction of the cartridge 10 at a time of replacing the holding portion 112 (or the cartridge unit or the like). Specific examples thereof include, but not particularly limited to, a claw, a pin, a hook, a clamp, a slide engagement, and the like, but a buckle type is preferable as illustrated in Fig. 28.

### (5) Operation example of odor generation device 1 of fifth embodiment

Hereinafter, an operation example of generating odor component-containing air from the odor generation device 1 of the fifth embodiment will be described.

First, the cartridge 10 is attached to the odor generation device 1. In a case where the number of the cartridges 10 is small, it is also possible to attach one each to a predetermined place. In a case where the number of cartridges 10 to be attached is large, a plurality of cartridges 10 can be attached to the odor generation device 1 at a time by using a unit-type cartridge holding unit 11 (that is, the cartridge unit) including the holding portion 112 that holds the plurality of cartridges 10 in advance as illustrated in Fig. 28.

Next, for example, in a case where a plurality of cartridges 10 is attached to the odor generation device 1, when an instruction to generate odor component-containing air is received from the outside, the arrangement drive unit drives the cartridge holding unit 11 to arrange the cartridge 10 having a target odor component near the release hole 120.

Next, a pusher is moved in a direction of the cartridge 10 by the drive mechanism unit connected to the cartridge 10 having the target odor component. When the operation shaft connected to the pusher moves in the direction of the cartridge 10, an inflow ventilation opening 30a opens, and air flows into a first ventilation portion 102a. When air flows into the cartridge 10, odor component-containing air is generated in the cartridge 10. Since the generation of odor component-containing air is as described above, a description thereof is omitted here.

Then, the odor component-containing air released from the cartridge 10 is released from the release hole 120 via the discharge hole 110 of the cartridge holding unit 11, and guided to the vicinity of the nose of the user.

### (6) Usage example of odor generation device 1 of fifth embodiment

A usage example of the odor generation device 1 is similar to that of the fourth embodiment, and thus a description thereof is omitted here.

Note that the present technology can have the following configurations.
[1] A cartridge for an odor generation device, the cartridge including:
   an odor retaining portion configured to retain an odor component;
   a ventilation portion having a ventilation opening that is openable and closable; and
   a connecting portion including at least two connection openings connecting the ventilation portion and the odor retaining portion, and a partition disposed upstream of one connection opening among the at least two connection openings.
[2] The cartridge for an odor generation device according to [1], in which
   the connecting portion further includes a shaft to push in from an upstream side, and
   the partition does not hinder an operation of the shaft.
[3] The cartridge for an odor generation device according to [1], in which
   the at least two connection openings include a first connection opening through which air is released from the connecting portion to the odor retaining portion, and a second connection opening through which odor component-containing air from the odor retaining portion is released to the connecting portion, and
   a diameter of the first connection opening is smaller than a diameter of the second connection opening.
[4] The cartridge for an odor generation device according to [1], in which the odor retaining portion includes an impregnant that retains an odor component.
[5] The cartridge for an odor generation device according to [4], in which the impregnant has a spiral structure in at least a part.
[6] The cartridge for an odor generation device according to [4], in which
   the odor retaining portion further includes a container portion, and
   the container portion includes an inner layer portion forming an inner side for holding the impregnant, and an outer layer portion forming an outer side of the container portion.
[7] The cartridge for an odor generation device according to [4], in which a length of the impregnant in a direction orthogonal to the connecting portion is longer than a length of the inner layer portion in a direction orthogonal to the connecting portion.
[8] The cartridge for an odor generation device according to [6] or [7], in which a part or all of the inner layer portion is formed containing glass or metal.
[9] The cartridge for an odor generation device according to [6], in which the outer layer portion includes a first fitting portion that is fitted to the connecting portion.
[10] The cartridge for an odor generation device according to [6], in which
   the ventilation portion includes an inner layer member that stores an opening and closing mechanism, and an outer layer member forming an outer side of the ventilation portion, and
   the inner layer member includes a second fitting portion in a fitting region between the outer layer member and the connecting portion.
[11] The cartridge for an odor generation device according to [10], in which a part of the outer layer member has a protrusion that prevents reverse assembly.
[12] The cartridge for an odor generation device according to any one of [1] to [11], in which
   the odor retaining portion further includes a lid, and
   the lid has an opening at a position corresponding to the connection opening.
[13] The cartridge for an odor generation device according to [6], in which the odor retaining portion further includes a fixing portion that fixes movement of the inner layer portion.
[14] The cartridge for an odor generation device according to any one of [1] to [13], in which at least a part of an outer periphery of the connecting portion has a protrusion that prevents reverse assembly.
[15] The cartridge for an odor generation device according to [10], in which knurling is applied to at least a part of an outer periphery of the outer layer portion and/or the outer layer member.
[16] The cartridge for an odor generation device according to any one of [1] to [15], further including:
   a releasing portion configured to release odor component-containing air to an outside, in which
   the releasing portion has a nozzle structure capable of changing a direction of odor component-containing air.
[17] An odor generation device including:
   a cartridge for an odor generation device, the cartridge including
   an odor retaining portion configured to retain an odor component,
   a ventilation portion having a ventilation opening that is openable and closable, and
   a connecting portion including at least two connection openings connecting the ventilation portion and the odor retaining portion, and a partition disposed upstream of one connection opening among the at least two connection openings; and
   a cartridge holding unit configured to hold the cartridge for an odor generation device.
[18] The odor generation device according to [17], in which
   the cartridge holding unit includes an indwelling portion that allows indwelling of the cartridge and has a discharge hole to discharge odor component-containing air released from the cartridge, and
   the indwelling portion includes a nozzle-shaped portion that discharges an airflow to a desired position, on the cartridge side.
[19] An olfactory examination device including:
   a cartridge for an odor generation device, the cartridge including
   an odor retaining portion configured to retain an odor component,
   a ventilation portion having a ventilation opening that is openable and closable, and
   a connecting portion including at least two connection openings connecting the ventilation portion and the odor retaining portion, and a partition disposed upstream of one connection opening among the at least two connection openings; and
   a cartridge holding unit configured to hold the cartridge for an odor generation device.

### REFERENCE SIGNS LIST

- 10: Cartridge for odor generation device
- 101: Odor retaining portion
- 102: Ventilation portion
- 102a: First ventilation portion
- 102b: Second ventilation portion
- 103: Connecting portion
- 104: Releasing portion
- 1: Odor generation device
- 11: Cartridge holding unit
- 110: Discharge hole
- 1101: Nozzle-shaped portion
- 111: Indwelling portion
- 112: Holding portion
- 113: Deodorization unit
- 12: Front storage portion
- 120: Release hole
- 121: Guide unit
- 13: Back storage portion
- 130: Fitting portion
- 20: Impregnant
- 21: Container portion
- 22: Lid
- 221: Opening
- 222: Rib
- 23: Fixing portion
- 24: Tubular body
- 211: Inner layer portion
- 212: Outer layer portion
- 2121: First fitting portion
- 30, 30a, 30b: Connection opening
- 31: Inner layer member
- 32: Outer layer member
- 311: Second fitting portion
- 321: Second fitted portion
- 322, 2122: Third fitting portion
- 40, 40a, 40b: Connection opening
- 41: Partition
- 42: First fitted portion
- 50: Nozzle structure
- 51: Locking portion
- 61: Protrusion
- 62: Knurling processing
- 63: Hooking portion
- X: Pusher
- Y: Jig
- L: Odor component

## Claims

1. A cartridge for an odor generation device, the cartridge comprising:
an odor retaining portion configured to retain an odor component;
a ventilation portion having a ventilation opening that is openable and closable; and
a connecting portion including at least two connection openings connecting the ventilation portion and the odor retaining portion, and a partition disposed upstream of one connection opening among the at least two connection openings.

2. The cartridge for an odor generation device according to claim 1, wherein
the connecting portion further includes a shaft to push in from an upstream side, and
the partition does not hinder an operation of the shaft.

3. The cartridge for an odor generation device according to claim 1, wherein
the at least two connection openings include a first connection opening through which air is released from the connecting portion to the odor retaining portion, and a second connection opening through which odor component-containing air from the odor retaining portion is released to the connecting portion, and
a diameter of the first connection opening is smaller than a diameter of the second connection opening.

4. The cartridge for an odor generation device according to claim 1, wherein the odor retaining portion includes an impregnant that retains an odor component.

5. The cartridge for an odor generation device according to claim 4, wherein the impregnant has a spiral structure in at least a part.

6. The cartridge for an odor generation device according to claim 4, wherein
the odor retaining portion further includes a container portion, and
the container portion includes an inner layer portion forming an inner side for holding the impregnant, and an outer layer portion forming an outer side of the container portion.

7. The cartridge for an odor generation device according to claim 4, wherein a length of the impregnant in a direction orthogonal to the connecting portion is longer than a length of the inner layer portion in a direction orthogonal to the connecting portion.

8. The cartridge for an odor generation device according to claim 6, wherein a part or all of the inner layer portion is formed containing glass or metal.

9. The cartridge for an odor generation device according to claim 6, wherein the outer layer portion includes a first fitting portion that is fitted to the connecting portion.

10. The cartridge for an odor generation device according to claim 6, wherein
the ventilation portion includes an inner layer member that stores an opening and closing mechanism, and an outer layer member forming an outer side of the ventilation portion, and
the inner layer member includes a second fitting portion in a fitting region between the outer layer member and the connecting portion.

11. The cartridge for an odor generation device according to claim 10, wherein a part of the outer layer member has a protrusion that prevents reverse assembly.

12. The cartridge for an odor generation device according to claim 1, wherein
the odor retaining portion further includes a lid, and
the lid has an opening at a position corresponding to the connection opening.

13. The cartridge for an odor generation device according to claim 6, wherein the odor retaining portion further includes a fixing portion that fixes movement of the inner layer portion.

14. The cartridge for an odor generation device according to claim 1, wherein at least a part of an outer periphery of the connecting portion has a protrusion that prevents reverse assembly.

15. The cartridge for an odor generation device according to claim 10, wherein knurling is applied to at least a part of an outer periphery of the outer layer portion and/or the outer layer member.

16. The cartridge for an odor generation device according to claim 1, further including:
a releasing portion configured to release odor component-containing air to an outside, wherein
the releasing portion has a nozzle structure capable of changing a direction of odor component-containing air.

17. An odor generation device comprising:
a cartridge for an odor generation device, the cartridge including
an odor retaining portion configured to retain an odor component,
a ventilation portion having a ventilation opening that is openable and closable, and
a connecting portion including at least two connection openings connecting the ventilation portion and the odor retaining portion, and a partition disposed upstream of one connection opening among the at least two connection openings; and
a cartridge holding unit configured to hold the cartridge for an odor generation device.

18. The odor generation device according to claim 17, wherein
the cartridge holding unit includes an indwelling portion that allows indwelling of the cartridge and has a discharge hole to discharge odor component-containing air released from the cartridge, and
the indwelling portion includes a nozzle-shaped portion that discharges an airflow to a desired position, on the cartridge side.

19. An olfactory examination device comprising:
a cartridge for an odor generation device, the cartridge including
an odor retaining portion configured to retain an odor component,
a ventilation portion having a ventilation opening that is openable and closable, and
a connecting portion including at least two connection openings connecting the ventilation portion and the odor retaining portion, and a partition disposed upstream of one connection opening among the at least two connection openings; and
a cartridge holding unit configured to hold the cartridge for an odor generation device.
